# EUROPÄISCHE PATENTSCHRIFT

(11) **EP 2 220 040 B1**
(45) Veröffentlichungstag und Bekanntmachung des Hinweises auf die Patenterteilung: **16.01.2013**
(21) Anmeldenummer: 08850642.3
(22) Anmeldetag: 06.11.2008
(51) Int. Cl.: C07D 205/08, A61K 31/397, A61P 3/06

(54) **NEUE KRISTALLINE DIPHENYLAZETIDINONHYDRATE, DIESE VERBINDUNGEN ENTHALTENDE ARZNEIMITTEL UND DEREN VERWENDUNG**
NEW CRYSTALLINE DIPHENYL ACETIDINONE HYDRATE, PHARMACEUTICAL AGENT CONTAINING SAID COMPOUND AND USE THEREOF
NOUVEAUX HYDRATES CRISTALLISÉS DE DIPHÉNYLAZÉTIDINONE, MÉDICAMENTS RENFERMANT CES COMPOSÉS, ET LEUR UTILISATION

(30) Priorität: 13.11.2007 DE 102007054497; 26.11.2007 DE 602007990255 T
(43) Veröffentlichungstag der Anmeldung: 25.08.2010
(73) Patentinhaber: Sanofi-Aventis Deutschland GmbH, 65929 Frankfurt am Main (DE)
(72) Erfinder: WOLLMANN, Theodor Andreas, 65926 Frankfurt am Main (DE); DUFFY, Regina, 65926 Frankfurt am Main (DE); CULLMANN, Frank, 65926 Frankfurt am Main (DE)
(86) Internationale Anmeldenummer: PCT/EP2008/009323
(87) Internationale Veröffentlichungsnummer: WO 2009/062619

(56) Entgegenhaltungen:
- US-A1- 2005 020 563
- US-B1- 7 067 689

## Beschreibung

Die Erfindung betrifft die kristallinen Hydrate eines substituierten Diphenylazetidinons

Amorphe Diphenylazetidinone sind bereits in US 7,205, 290 beschrieben. Zu dieser Zeit waren keine Hydrate dieser Diphenylazetidinone bekannt.

Der Erfindung lag die Aufgabe zugrunde, ein Diphenylazetidinon zur Verfugung zu stellen, das im Vergleich mit den in US 7,205, 290 beschriebenen, verbesserte Eigenschaften aufweist. Weiter bestand die Aufgabe in einer Erhöhung der Lösungsgeschwindigkeit der amorphen Diphenylazetidinone aus US 7,205, 290.

Die Lösung der Aufgabe besteht in der Bereitstellung von kristallinen Hydraten der Formel I 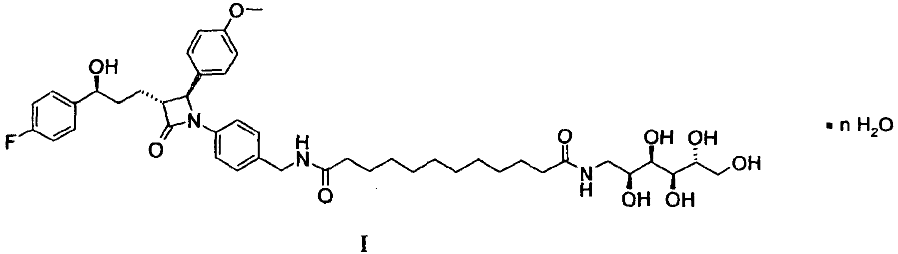 worin n einen Wert von 0,5 bis 1,8 besitzt.

Bevorzugt sind kristalline Hydrate der Verbindung der Formel I, worin n einen Wert von 0,8 bis 1,3 besitzt.

Bevorzugt sind kristalline Hydrate der Verbindung der Formel I, worin n einen Wert von 1,0 bis 1,2 besitzt.

Durch die Bereitstellung der erfindungsgemäßen kristallinen Hydrate der Formel I kann der Wirkstoff
- leichter gereinigt werden (z.B. durch Umkristallisieren)
- eine für die Arzneimittelzulassung nötige definierte Reinheit aufweisen
- leicht durch gängige Methoden wie XRPD (X-ray Powder Diffraction), Schmelzpunkt, IR (Infrarot Spektrum) nachgewiesen und identifiziert werden und er besitzt
- eine reproduzierbare physikalische Qualität
- eine bestimmte definierte Teilchengröße und
- eine spezielle definierte Oberfläche

Kristalline Wirkstoffe sind in der Regel stabiler als amorphe. Dadurch werden Probleme mit dem Abbau der Wirkstoffe und den auftretenden Abbauprodukten vermieden.

Die amorphe Form eines Wirkstoffs kann noch einen ungewünschten Gehalt an Lösungsmitteln enthalten. Diese sind in der Regel schwer zu entfernen, da nicht umkristallisiert werden kann.

Die amorphe Form ist energiereicher als die kristalline Form. Das kann dazu führen, dass sich das zufällige Muster der Molekülverteilung der amorphen Form spontan unter Energieabgabe rearrangiert und einen Teil der Energie abführt. Dies kann zu einer Änderung der Wirkung des Wirkstoffs führen, ohne dass dies direkt an einem messbaren Parameter des Wirkstoffs sichtbar würde. Ein signifikante Einfluss auf die Zuverlässigkeit des Wirkstoffs und somit ein Risiko für den Patienten ist die Folge.

Es ist schwer zu beweisen, dass unterschiedliche Chargen des amorphen Wirkstoffs ^gleichartig sind.

Eine weitere Ausführungsform der Erfindung umfasst kristallines Hydrat der Formel I, dadurch gekennzeichnet, dass das mit CuKα-Strahlung gemessene XRPD einen Hauptpeak von 20.83 Grad 2 Theta ± 0.2 Grad 2 Theta aufweist.

Eine weitere Ausführungsform der Erfindung umfasst kristallines Hydrat der Formel I, dadurch gekennzeichnet, dass das mit CuKα-Strahlung gemessene XRPD zumindest Peaks von folgenden 2 Theta Werten aufweist:
20.83 und 21.58 ± 0.2 Grad 2 Theta.

Eine weitere Ausführungsform der Erfindung umfasst kristallines Hydrat der Formel I, dadurch gekennzeichnet, dass das mit CuKα-Strahlung gemessene XRPD zumindest Peaks von folgenden 2 Theta Werten aufweist:
12.19, 20.83 und 21.58 ± 0.2 Grad 2 Theta.

Eine weitere Ausführungsform der Erfindung umfasst kristallines Hydrat der Formel I, dadurch gekennzeichnet, dass das mit CuKα-Strahlung gemessene XRPD zumindest Peaks von folgenden 2 Theta Werten aufweist:
20.43, 20.83, 21.58 ± 0.2 Grad 2 Theta.

Eine weitere Ausführungsform der Erfindung umfasst kristallines Hydrat der Formel I, dadurch gekennzeichnet, dass das mit CuKα-Strahlung gemessene XRPD zumindest Peaks von folgenden 2 Theta Werten aufweist:
12.19, 17.31, 20.43, 20.83, 21.58 ± 0.2 Grad 2 Theta.

Eine weitere Ausführungsform der Erfindung umfasst kristallines Hydrat der Formel I, dadurch gekennzeichnet, dass das mit CuKα-Strahlung gemessene XRPD zumindest Peaks von folgenden 2 Theta Werten aufweist:
7.33, 12.19, 16.05, 17.31, 20.83, 21.58 ± 0.2 Grad 2 Theta.

Eine weitere Ausführungsform der Erfindung umfasst kristallines Hydrat der Formel I, dadurch gekennzeichnet, dass das mit CuKα-Strahlung gemessene XRPD zumindest Peaks von folgenden 2 Theta Werten aufweist:
7.33, 8.92, 12.19, 16.05, 17.31, 17.68, 18.83, 20.43, 20.83, 21.58, 24.55, 25.37 ± 0.2 Grad 2 Theta.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,01 mg bis 100 mg (typischerweise von 0,05 mg und 50 mg) pro Tag pro Kilogramm Körpergewicht, z.B. 0,05-10 mg/kg/Tag.

Oral verabreichbare Einzeldosisformulierungen, wie zum Beispiel Tabletten oder Kapseln, können beispielsweise von 1,0 bis 1000 mg, typischerweise von 5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Möglich sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteter Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Die erfindungsgemäßen Verbindung(en) der Formel (I) können auch in Kombination mit weiteren Wirkstoffen verabreicht werden.

Als weitere Wirkstoffe für die Kombinationspräparate sind geeignet:
Alle Antidiabetika, die in der Roten Liste 2007, Kapitel 12 genannt sind; alle Abmagerungsmittel/Appetitzügler, die in der Roten Liste 2007, Kapitel 1 genannt sind; alle Diuretika, die in der Roten Liste 2007, Kapitel 36 genannt sind; alle Lipidsenker, die in der Roten Liste 2007, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Erfolgt die Gabe der Wirkstoffe durch getrennte Verabreichung der Wirkstoffe, so kann diese gleichzeitig oder nacheinander erfolgen.
Die meisten der nachfolgend aufgeführten Wirkstoffe sind in USP Dictionary of USAN and International Drug Names, US Pharmacopeia, Rockville 2006, offenbart.

Antidiabetika umfassen Insulin und Insulinderivate, wie z.B. Lantus^{®} (siehe www.lantus.com) oder HMR 1964 oder Levemir® (insulin detemir), Humalog^{(R)} (Insulin Lispro), Humulin^{(R)}, VIAject™, SuliXen^{(R)} oder solche, wie sie in W02005005477 (Novo Nordisk) beschrieben sind, schnell wirkende Insuline (siehe US 6,221,633), inhalierbare Insuline, wie z. B. Exubera^{®} , Nasulin™, oder orale Insuline, wie z. B. IN-105 (Nobex) oder Oral-lyn ™ (Generex Biotechnology) oder Technosphere^{(R)} Insulin (MannKind) oder Cobalamin™ orales Insulin oder Insuline, wie sie in W02007128815, WO2007128817, WO2008034881, WO2008049711 beschrieben sind oder Insuline, die transdermal verabreicht werden können;

GLP-1-Derivate und GLP-1 Agonisten wie z.B. Exenatide oder spezielle Zubereitungen davon, wie sie z.B. in WO2008061355 beschrieben sind, Liraglutide, Taspoglutide oder diejenigen die in WO 98/08871, W02005027978, W02006037811, W02006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), AVE-0010, BIM-51077 (R-1583, ITM-077), PC-DAC:Exendin-4 (ein Exendin-4 Analogon, welches kovalent an rekombinantes menschliches Albumin gebunden ist), CVX-73, CVX-98 und CVx-96 (GLP-1 Analoga, welche kovalent an einen monoklonalen Antikörper gebunden sind, der spezifische Bindungsstellen für das GLP-1 Peptid aufweist), CNTO-736 (ein GLP-1 Analogon, welches an eine Domäne gebunden ist, welche den Fc-Teil eines Antikörpers beinhaltet), PGC-GLP-1 (GLP-1 gebunden an einen Nanocarrier), Agonisten wie sie z.B. bei D. Chen et al., Proc. Natl. Acad. Sci. USA 104 (2007) 943 beschrieben sind, solche wie sie in WO2006124529, WO2007124461 beschrieben sind, Peptide wie z.B. Obinepitide (TM-30338), Amylinrezeptor Agonisten, wie sie z.B. in W02007104789 beschrieben sind, Analoga des humanen GLP-1, wie sie in W02007120899, WO2008022015, W02008056726 beschrieben sind, sowie oral wirksame hypoglykämische Wirkstoffe.

Antidiabetika umfassen auch Agonisten des Glukose-abhängigen insulinotropen Polypeptids (GIP) Rezeptors wie sie z.B. in W02006121860 beschrieben sind.

Antidiabetika umfassen auch das Glukose-abhängige insulinotrope Polypeptid (GIP) wie auch analoge Verbindungen wie sie z.B. in WO2008021560 beschrieben sind.

Antidiabetika umfassen auch Analoga und Derivate des Fibroblastenwachstumsfaktors 21 (FGF-21, fibroblast growth factor 21).

Die oral wirksamen hypoglykämischen Wirkstoffe umfassen vorzugsweise Sulfonylharnstoffe, Biguanidine,
Meglitinide,
Oxadiazolidindione,
Thiazolidindione,
PPAR- und RXR-Modulatoren,
Glukosidase-Inhibitoren,
Hemmstoffe der Glykogenphosphorylase,
Glukagonrezeptor-Antagonisten,
Glukokinaseaktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT),
GLP-1-Agonisten,
Kaliumkanalöffner, wie z.B. Pinacidil, Cromakalim, Diazoxid oder solche wie sie bei R. D. Carr et al., Diabetes 52, 2003, 2513.2518, bei J. B. Hansen et al, Current Medicinal Chemistry 11, 2004, 1595-1615, bei T. M. Tagmose et al., J. Med. Chem. 47, 2004, 3202-3211 oder bei M. J. Coghlan et al., J. Med. Chem. 44, 2001, 1627-1653 beschrieben sind, oder diejenigen, die in WO 97/26265 und WO 99/03861 von Novo Nordisk A/S offenbart wurden,
Wirkstoffe, die auf den ATP-abhängigen Kaliumkanal der Betazellen wirken,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption, Modulatoren der natrium-abhängigen Glukosetransporter 1 oder 2 (SGLT1, SGLT2), Hemmstoffe der 11-beta-Hydroxysteroid-Dehydrogenase-1 (11ß-HSD1),
Inhibitoren der Protein-Tyrosin-Phosphatase-1B (PTP-1B),
Nikotinsäurerezeptoragonisten,
Inhibitoren der hormon-sensitiven bzw. endothelialen Lipasen,
Hemmstoffen der Acetyl-CoA Carboxylase (ACC1 und/oder ACC2) oder
Inhibitoren der GSK-3 beta.

Weiterhin sind umfasst den Fettstoffwechsel verändernde Verbindungen wie antihyperlipidämische Wirkstoffe und antilipidämische Wirkstoffe,
HMGCoA-Reduktase-Inhibitoren,
Farnesoid X Rezeptor (FXR) Modulatoren,
Fibrate,
Cholesterinresreptionsinhibitoren,
CETP-Inhibitoren,
Gallensäureresorptionsinhibitoren,
MTP-Inhibitoren;
Agonisten des Estrogenrezeptors gamma (ERR□ Agonisten),
Sigma-1 Rezeptorantagonisten,
Antagonisten des Somatostatin 5 Rezeptors (SST5 Rezeptor);
Verbindungen, die die Nahrungsmitteleinnahme verringern und
Verbindungen, die die Thermogenese erhöhen.

Bei einer Ausführungsform der Erfindung wird die Verbindung der Formel I in Kombination mit Insulin verabreicht.

Bei einer Ausführungsform wird die Verbindung der Formel I in Kombination mit einem Wirkstoff, der auf den ATP-abhängigen Kaliumkanal der Betazellen wirkt, z.B. Sulfonylharnstoffe, wie z.B. Tolbutamid, Glibenclamid, Glipizid, Gliclazide oder Glimepirid, verabreicht.

Bei einer Ausführungsform wird die Verbindung der Formel I in Kombination mit einer Tablette verabreicht, die sowohl Glimeprid enthält, welches schnell freigesetzt wird wie auch Metformin enthält, welches über einen längeren Zeitraum freigesetzt wird (wie z.B. in US2007264331, WO2008050987 beschrieben).

Bei einer Ausführungsform wird die Verbindung der Formel I in Kombination mit einem Biguanid, wie z.B. Metformin, verabreicht.

Bei wieder einer Ausfühnmgsform wird die Verbindung der Formel I in Kombination mit einem Meglitinid, wie z.B. Repaglinide, Nateglinid oder Mitiglinide verabreicht.

Bei einer weiteren Ausführungsform wird die Verbindung der Formel I mit einer Kombination von Mitiglinide mit einem Glitazon, z.B. Pioglitazon Hydrochlorid, verabreicht.

Bei einer weiteren Ausführungsform wird die Verbindung der Formel I mit einer Kombination von Mitiglinide mit einem alpha-Glukosidaseinhibitor verabreicht.

Bei einer weiteren Ausführungsform wird die Verbindung der Formel I in Kombination mit antidiabetischen Verbindungen, wie sie in WO2007095462, WO200710106 WO2007105650 beschrieben sind, verabreicht.

Bei einer weiteren Ausführungsform wird die Verbindung der Formel I in Kombination mit antihypoglykämischen Verbindungen, wie sie in W02007137008 beschrieben sind, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon), DRL-17564, DRF-2593 (Balaglitazon), INT-131, T-2384 oder solchen, wie sie in WO2005086904, WO2007060992, WO2007100027, WO2007103252, W02007122970, W02007138485, W02008006319, WO2008006969, W02008010238, W02008017398, WO2008028188 beschrieben sind, verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit Tandemact™, einer festen Kombination von Pioglitazon mit Glimeprid, verabreicht.

Bei einer weiteren Ausführungsform der Erfindung wird die Verbindung der Formel I in Kombination mit einer festen Kombination von Pioglitazon Hydrochlorid mit einem Angiotensin II Agonisten, wie z.B. TAK-536, verabreicht.

Bei einer Ausführungsform der Erfindung wird die Verbindung der Formel I in Kombination mit einem PPAR alpha Agonisten bzw. gemischten PPAR alpha/PPAR delta Agonisten, wie z.B. GW9578, GW-590735, K-111, LY-674, KRP-101, DRF-10945, LY-518674, CP-900691, BMS-687453, BMS-711939 oder solchen wie sie in WO2001040207, W02002096894, WO2005097076, WO2007056771, WO2007087448, WO2007089667, W02007089557, W02007102515, WO2007103252, JP2007246474, W02007118963, W02007118964, WO2007126043, WO2008006043, WO2008006044, WO2008012470, W02008035359 beschrieben sind, verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, CKD-501 (Lobeglitazon Sulfat), MBX-213, KY-201 oder wie in WO 00/64888, WO 00/64876, WO03/020269, WO2004024726, WO2007099553, US2007276041, WO2007085135, W02007085136, WO2007141423, W02008016175, WO2008053331 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 oder wie sie in WO2006059744, WO2006084176, WO2006029699, WO2007039172-WO2007039178, W02007071766, W02007101864, US2007244094, W02007119887, W02007141423, US2008004281, W02008016175 beschrieben sind, verabreicht.

Bei einer Ausführungsform der Erfindung wird die Verbindung der Formel I in Kombination mit einem pan-SPPARM (selective PPAR modulator alpha, gamma, delta), wie z.B. GFT-505 oder solchen wie sie in WO2008035359 beschrieben sind, verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht.

Bei einer Ausführungsform wird die Verbindung der Formel I in Kombination mit einem α-Glukosidase-Inhibitor, wie z.B. Miglitol oder Acarbose oder solchen, wie sie z.B. in W02007114532, W02007140230, US2007287674, US2008103201 beschrieben sind, verabreicht.

Bei einer Ausführungsform wird die Verbindung der Formel I in Kombination mit einem Hemmstoff der Glykogenphosphorylase, wie z.B. PSN-357 oder FR-258900 oder solchen wie in W02003084922, WO2004007455, WO2005073229-31, WO2005067932 beschrieben, verabreicht.

Bei einer Ausführungsform wird die Verbindung der Formel I in Kombination mit GlukagonRezeptor-Antagonisten, wie z.B. A-770077 oder NNC-25-2504 oder wie in WO2004100875, WO2005065680, WO2006086488, WO2007047177, W02007106181, WO2007111864, W02007120270, W02007120284, WO2007123581, WO2007136577, W02008042223 beschrieben, verabreicht.

Bei einer weiteren Ausführungsform wird die Verbindung der Formel I in Kombination mit einer Antisense-Verbindung, z.B. ISIS-325568, verabreicht, welche die Produktion des Glukagonrezeptors inhibiert.

Bei einer Ausführungsform wird die Verbindung der Formel I in Kombination mit Aktivatoren der Glukokinase, wie z. B. LY-2121260 (W02004063179), PSN-105, PSN-110, GKA-50 oder solchen wie sie z. B. in WO2004072031, W02004072066, WO2005080360, WO2005044801, W02006016194, WO2006058923, W02006112549, WO2006125972, WO2007017549, WO2007017649, WO2007007910, W02007007040-42, WO2007006760-61, WO2007006814, W02007007886, W02007028135, WO2007031739, W02007041365, W02007041366, W02007037534, WO2007043638, WO2007053345, WO2007051846, WO2007051845, W02007053765, W02007051847, WO2007061923, WO2007075847, WO2007089512, WO2007104034, W02007117381, WO2007122482, WO2007125103, W02007125105, US2007281942, WO2008005914, W02008005964, WO2008043701, WO2008044777, W02008047821, US2008096877, W02008050117, WO2008050101, W02008059625 beschrieben sind, verabreicht.

Bei einer Ausführungsform wird die Verbindung der Formel I in Kombination mit einem Inhibitor der Glukoneogenese, wie sie z. B. in FR-225654, WO2008053446 beschrieben sind, verabreicht.

Bei einer Ausführungsform wird die Verbindung der Formel I in Kombination mit Inhibitoren der Fructose-1,6-bisphosphatase (FBPase) wie z.B. MB-07729, CS-917 (MB-06322) oder MB-07803 oder solchen wie sie in WO2006023515, W02006104030, WO2007014619, W02007137962, WO2008019309, WO2008037628 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in W02004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Sitagliptin Phosphat, Saxagliptin ((BMS-477118), GSK-823093, PSN-9301, SYR-322, SYR-619, TA-6666, TS-021, GRC-8200 (Melogliptin), GW-825964X, KRP-104, DP-893, ABT-341, ABT-279 oder ein anderes Salz davon, S-40010, S-40755, PF-00734200, BI-1356, PHX-1149, Alogliptin oder solchen Verbindungen wie sie in WO2003074500, WO2003106456, WO2004037169, WO200450658, WO2005037828, WO2005058901, WO2005012312, WO2005/012308, WO2006039325, WO2006058064, WO2006015691, WO2006015701, WO2006015699, WO2006015700, W02006018117, WO2006099943, WO2006099941, JP2006160733, WO2006071752, WO2006065826, WO2006078676, WO2006073167, WO2006068163, WO2006085685, WO2006090915, WO2006104356, W02006127530, W02006111261, US2006890898, US2006803357, US2006303661, WO2007015767 (LY-2463665), WO2007024993, WO2007029086, WO2007063928, WO2007070434, WO2007071738, WO2007071576, WO2007077508, WO2007087231, WO2007097931, W02007099385, WO2007100374, WO2007112347, WO2007112669, WO2007113226, W02007113634, W02007115821, W02007116092, US2007259900, EP1852108, US2007270492, WO2007126745, WO2007136603, W02007142253, WO2007148185, W02008017670, US2008051452, W02008027273, WO2008028662, W02008029217, JP2008031064, JP2008063256, W02008033851, WO2008040974, W02008040995, W02008064107 beschrieben sind, verabreicht.

Bei einer Ausführungsform wird die Verbindung der Formel I in Kombination mit Janumet™, einer festen Kombination von Sitagliptin Phosphat mit Metformin Hydrochlorid, verabreicht.

Bei einer Ausführungsform wird die Verbindung der Formel I in Kombination mit Eucreas^{(R)}, einer festen Kombination von Vildagliptin mit Metformin Hydrochlorid, verabreicht.

Bei einer Ausführungsform wird die Verbindung der Formel I in Kombination mit einer festen Kombination von eines Salzes von Sitagliptin mit Metformin Hydrochlorid, verabreicht.

Bei einer Ausführungsform wird die Verbindung der Formel I in Kombination mit einer Kombination eines DPP-IV-Inhibitors mit omega-3-Fettsäuren oder omega-3-Fettsäureestern, wie z.B. in WO2007128801 beschrieben, verabreicht.

Bei einer Ausführungsform wird die Verbindung der Formel I in Kombination mit einer die Insulinsekretion verstärkende Substanz, wie z. B. KCP-265 (WO2003097064), oder solchen wie sie in WO2007026761, WO2008045484 beschrieben sind, verabreicht.

Bei einer Ausführungsforin wird die Verbindung der Formel I in Kombination mit Agonisten des glucose-abhängigen insulinotropischen Rezeptors (GDIR) wie z. B. APD-668 verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268, SAR 7226, SGL-5083, SGL-5085, SGL-5094, ISIS-388626, Sergliflozin oder Dapagliflozin oder wie sie z. B. in WO2004007517, WO200452903, W0200452902, PCT/EP2005/005959, W02005085237, JP2004359630, WO2005121161, WO2006018150, W02006035796, W02006062224, WO2006058597, W02006073197, W02006080577, WO20060879979, WO2006108842, WO2007000445, W02007014895, W02007080170, W02007093610, W02007126117, WO2007128480, W02007129668, US2007275907, WO2007136116, WO2007143316, WO2007147478, W02008001864, WO2008002824, WO2008013277, WO2008013280, WO2008013321, WO2008013322, W02008016132, WO2008020011, JP2008031161, W02008034859, W02008042688, W02008044762, WO2008046497, WO2008049923, W02008055870, WO2008055940 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Hemmstoffen der 11-beta-Hydroxysteroid-Dehydrogenase- (11ß-HSD1), wie z. B. BVT-2733, JNJ-25918646, INCB-13739, INCB-20817, DIO-92 ((-)-Ketoconazol) oder solche, wie sie z. B. in WO200190090-94, WO200343999, WO2004112782, W0200344000, WO200344009, W02004112779, W02004113310, WO2004103980, WO2004112784, W02003065983, W02003104207, WO2003104208, WO2004106294, WO2004011410, W02004033427, WO2004041264, WO2004037251, WO2004056744, WO2004058730, W02004065351, W02004089367, WO2004089380, WO2004089470-71, WO2004089896, WO2005016877, WO2005063247, W02005097759, WO2006010546, WO2006012227, W02006012173, WO2006017542, WO2006034804, WO2006040329, W02006051662, W02006048750, W02006049952, WO2006048331, WO2006050908, WO2006024627, WO2006040329, W02006066109, W02006074244, WO2006078006, WO2006106423, WO2006132436, W02006134481, WO2006134467, WO2006135795, W02006136502, W02006138508, W02006138695, WO2006133926, WO2007003521, WO2007007688, US2007066584, WO2007029021, WO2007047625, WO2007051811, WO2007051810, WO2007057768, W02007058346, WO2007061661, W02007068330, WO2007070506, WO2007087150, W02007092435, WO2007089683, WO2007101270, WO2007105753, WO2007107470, W02007107550, W02007111921, US2007207985, US2007208001, WO2007115935, W02007118185, W02007122411, WO2007124329, WO2007124337, WO2007124254, W02007127688, WO2007127693, WO2007127704, WO2007127726, WO2007127763, W2007127765, W02007127901, US2007270424, JP2007291075, WO2007130898, WO2007135427, W02007139992, W02007144394, WO2007145834. W02007145835, WO2007146761, W02008000950, W02008000951, W02008003611, W02008005910, WO2008006702, W02008006703, WO2008011453, W02008012532, W02008024497, WO2008024892, W02008032164, WO2008034032, WO2008043544, WO2008044656, WO2008046758, W02008052638, WO2008053194 beschrieben sind, verabreicht.

Bei einer Ausführungsform wird die Verbindung der Formel I in Kombination mit Inhibitoren der Protein-Tyrosin-Phosphatase-1B (PTP-1B), wie sie z. B. in W0200119830-31, W0200117516, WO2004506446, WO2005012295, W02005116003, W02005116003, W02006007959, DE 10 2004 060542.4, WO2007009911, W02007028145, W02007067612-615, WO2007081755, W02007115058, US2008004325, WO2008033455, WO2008033931, W02008033932, W02008033934 beschrieben sind, verabreicht.

Bei einer Ausführungsform der Erfindung wird die Verbindung der Formel I in Kombination mit einem Agonisten des GPR109A (HM74A Rezeptor Agonisten; NAR-Agonisten (Nikotinsäurerezeptoragonisten)), wie z.B. Nicotinsäure oder "extended release niacin" in Verbindung mit MK-0524A (Laropiprant) oder MK-0524 oder solchen Verbindungen, wie sie in WO2004041274, WO2006045565, W02006045564, WO2006069242, W02006085108, W02006085112, WO2006085113; WO2006124490, W02006113150, W02007017261, W02007017262, WO2007017265, WO2007015744, W02007027532, WO2007092364, W02007120575, WO2007134986, WO2007150025, WO2007150026, WO2008016968, W02008051403 beschrieben sind, verabreicht.

Bei einer anderen Ausführungsform der Erfindung wird die Verbindung der Formel I in Kombination mit einer festen Kombination von Niacin mit Simvastatin verabreicht.

Bei einer anderen Ausführungsform der Erfindung wird die Verbindung der Formel I in Kombination mit Nicotinsäure oder "extended release niacin" in Verbindung mit MK-0524A (Laropiprant) verabreicht.

Bei einer weiteren Ausführungsform der Erfindung wird die Verbindung der Formel I in Kombination mit Nicotinsäure oder "extended release niacin" in Verbindung mit MK-0524A (Laropiprant) und mit Simvastatin verabreicht.

Bei einer Ausführungsform der Erfindung wird die Verbindung der Formel I in Kombination mit Nicotinsäure oder einem anderen Nicotinsäurerezeptoragonisten und einem Prostaglandin DP Rezeptorantagonisten, wie z.B. solchen wie sie in W02008039882 beschrieben sind, verabreicht.

Bei einer anderen Ausführungsform der Erfindung wird die Verbindung der Formel I in Kombination mit einem Agonisten des GPR116, wie sie z.B. in WO2006067531, W02006067532 beschrieben sind, verabreicht.

Bei einer Ausführungsform wird die Verbindung der Formel I in Kombination mit Modulatoren des GPR40, wie sie z.B. in WO2007013689, W02007033002, WO2007106469, US2007265332, WO2007123225, WO2007131619, W02007131620, WO2007131621, US2007265332, W02007131622, WO2007136572, WO2008001931, WO2008030520, W02008030618, W02008054674, WO2008054675 beschrieben sind, verabreicht.

Bei einer Ausführungsform wird die Verbindung der Formel I in Kombination mit Modulatoren des GPR119 (G-Protein-gekoppelter Glukose-abhängiger insulinotroper Rezeptor), wie z.B. PSN-119-1, MBX-2982 oder solchen wie sie z. B. in WO2005061489 (PSN-632408), WO2004065380, W02007003960-62 und WO2007003964, W02007116229, WO2007116230, W02008005569, WO2008005576, WO2008008887, WO2008008895, WO2008025798, WO2008025799, WO2008025800 beschrieben sind, verabreicht.

Bei einer weiteren Ausführungsform wird die Verbindung der Formel I in Kombination mit Modulatoren des GPR120, wie sie z.B. in EP1688138 beschrieben sind, verabreicht.

Bei einer Ausführungsform wird die Verbindung der Formel I in Kombination mit Inhibitoren der hormon-sensitiven Lipase (HSL) und/oder Phospholipasen, wie z. B. in WO2005073199, WO2006074957, W02006087309, W02006111321, WO2007042178, W02007119837 beschrieben, verabreicht.

Bei einer Ausführungsform wird die Verbindung der Formel I in Kombination mit Inhibitoren der endothelialen Lipase, wie z. B. in W02007110216 beschrieben, verabreicht.

Bei einer Ausführungsform wird die Verbindung der Formel I in Kombination mit einem Phospholipase A2 Inhibitor wie z.B. Darapladib oder A-002 oder solchen, wie sie in W02008048866, W020080488867 beschrieben sind, verabreicht.

Bei einer Ausführungsform wird die Verbindung der Formel I in Kombination mit Myricitrin, einem Lipase-Inhibitor (W02007119827), verabreicht.

Bei einer Ausführungsform wird die Verbindung der Formel I in Kombination mit einem Inhibitor der Glykogen Synthase Kinase-3 beta (GSK-3 beta), wie z. B. in US2005222220, W02005085230, W02005111018, WO2003078403, WO2004022544, W02003106410, W02005058908, US2005038023, WO2005009997, US2005026984, WO2005000836, WO2004106343, EP1460075, WO2004014910, WO2003076442, WO2005087727, W02004046117, W02007073117, WO2007083978, WO2007120102, W02007122634, WO2007125109, WO2007125110, US2007281949, WO2008002244, WO2008002245, WO2008016123, WO2008023239, WO2008044700, WO2008056266, WO2008057940 beschrieben.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Phosphoinositidkinase-3 (PI3K), wie z.B. solchen, wie in WO2008027584 beschrieben, verabreicht.

Bei einer Ausführungsform wird die Verbindung der Formel I in Kombination mit einem Inhibitor der Serum/Glucocorticoid regulierten Kinase (SGK), wie z. B. in WO2006072354, W02007093264, WO2008009335 beschrieben, verabreicht.

Bei einer Ausführungsform wird die Verbindung der Formel I in Kombination mit einem Modulator des Glucocorticoidrezeptors, wie z. B. in WO2008057855, WO2008057856, W02008057857, WO2008057859, WO2008057862 beschrieben, verabreicht.

Bei einer Ausführungsform wird die Verbindung der Formel I in Kombination mit einem Inhibitor der Protein Kinase C beta (PKC beta), wie z. B. Ruboxistaurin, verabreicht.

Bei einer weiteren Ausführungsform wird die Verbindung der Formel I in Kombination mit einem Aktivator der AMP-aktivierten Proteinkinase (AMPK), wie sie z. B. in WO2007062568, W02008006432, WO2008016278, WO2008016730 beschrieben sind, verabreicht.

Bei einer Ausführungsform wird die Verbindung der Formel I in Kombination mit einem Inhibitor der Ceramidkinase, wie sie z. B. in W02007112914, W02007149865 beschrieben sind, verabreicht.

Bei einer weiteren Ausführungsform wird die Verbindung der Formel I in Kombination mit einem Inhibitor der MAPK-interagierenden Kinase 1 oder 2 (MNK1 oder 2), wie sie z.B. in W02007104053, W02007115822, WO2008008547 beschrieben sind, verabreicht.

Bei einer Ausführungsform wird die Verbindung der Formel I in Kombination mit Inhibitoren der "I-kappaB kinase" (IKK Inhibitoren), wie sie z. B. in W02001000610, W02001030774, W02004022057, WO2004022553, WO2005097129, W02005113544, US2007244140 beschrieben sind, verabreicht.

Bei einer anderen Ausführungsform wird die Verbindung der Formel I in Kombination mit Inhibitoren der NF-kappaB (NFKB) Aktivierung, wie sie z. B. Salsalate verabreicht.

Bei einer weiteren Ausführungsform wird die Verbindung der Formel I in Kombination mit Inhibitoren der ASK-1 (apoptosis signal-regulating kinase 1), wie sie z. B. in W02008016131 beschrieben sind, verabreicht.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699, BMS-644950 oder solchen, wie sie in US2007249583 beschrieben sind, verabreicht.

Bei einer weiteren Ausführungsform der Erfindung wird die Verbindung der Formel I in Kombination mit einem Farnesoid X Rezeptor (FXR) Modulatoren, wie z.B. WAY-362450 oder solchen wie in WO2003099821, WO2005056554, WO2007052843, WO2007070796, WO2007092751, JP2007230909, W02007095174, WO2007140174, WO2007140183, WO2008000643, WO2008002573, W02008025539, W02008025540 beschrieben, verabreicht.

Bei einer anderen Ausführungsform der Erfindung wird die Verbindung der Formel I in Kombination mit einem Liganden des Leber X Rezeptors (liver X receptor; LXR), wie z.B. in WO2007092965, W02008041003, WO2008049047 beschrieben, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit Fibraten, wie z.B. dem Cholinsalz von Fenofibrat (SLV-348), verabreicht.

Bei einer Ausführungsform der Erfindung wird die Verbindung der Formel I in Kombination mit Fibraten, wie z.B. dem Cholinsalz von Fenofibrat und einem HMGCoA Reduktase Inhibitor, wie z.B. Rosuvastatin, verabreicht.

Bei einer weiteren Ausführungsform der Erfindung wird die Verbindung der Formel I in Kombination mit Bezafibrat und Diflunisal verabreicht.

Bei einer weiteren Ausführungsform der Erfindung wird die Verbindung der Formel I in Kombination mit einer festen Kombination von Fenofibrat oder einem Salz davon mit Simvastatin, Rosuvastatin, Fluvastatin, Lovastatin, Cerivastatin, Pravastatin oder Atorvastatin verabreicht.

Bei einer weiteren Ausführungsform der Erfindung wird die Verbindung der Formel I in Kombination mit Synordia (R), einer festen Kombination von Fenofibrat mit Metformin, verabreicht.

Bei einer Ausfühnmgsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, W02005005453), MD-0727 (Microbia Inc., WO2005021497, WO2005021495) oder mit Verbindungen, wie in WO2002066464, WO2005000353 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder W02005033100 (Lipideon Biotechnology AG) oder wie in WO2002050060, WO2002050068, WO2004000803, W02004000804, WO2004000805, WO2004087655, W02004097655, W02005047248, W02006086562, WO2006102674, W02006116499, WO2006121861, W02006122186, W02006122216, WO2006127893, WO2006137794, WO2006137796, W02006137782, W02006137793, WO2006137797, WO2006137795, WO2006137792, W02006138163, WO2007059871, US2007232688, WO2007126358, WO2008033431, WO2008033465, W02008052658, WO2008057336 beschrieben, verabreicht.

Bei einer Ausführungsform der Erfindung wird die Verbindung der Formel I in Kombination mit einem NPC1L1-Antagonisten, wie z.B. solchen, wie sie in WO2008033464, WO2008033465 beschrieben sind, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Atorvastatin, verabreicht.

Bei einer Ausführungsform der Erfindung wird die Verbindung der Formel I in Kombination mit einer festen Kombination von Ezetimibe mit Fenofibrat verabreicht.

Bei einer Ausführungsform der Erfindung ist der weitere Wirkstoff ein Diphenylazetidinonderivat, wie z.B. in US 6,992,067 oder US 7,205,290 beschrieben.

Bei einer weiteren Ausführungsform der Erfindung ist der weitere Wirkstoff ein Diphenylazetidinonderivat, wie z.B. in US 6,992,067 oder US 7,205,290 beschrieben, kombiniert mit einem Statin, wie z.B. Simvastatin, Fluvastatin, Pravastatin, Lovastatin, Cerivastatin, Atorvastatin oder Rosuvastatin.

Bei einer Ausführungsform der Erfindung wird die Verbindung der Formel I in Kombination mit einer festen Kombination von Lapaquistat, einem Squalensynthase-Inhibitor, mit Atorvastatin verabreicht.

Bei einer Ausführungsform der Erfindung wird die Verbindung der Formel I in Kombination mit einem CETP-Inhibitor, wie z.B. Torcetrapib, Anacetrapib oder JTT-705 (Dalcetrapib) oder solchen wie sie in WO2006002342, W02006010422, W02006012093, WO2006073973, W02006072362, WO2007088996, WO2007088999, US2007185058, US2007185113, US2007185154, US2007185182, WO2006097169, WO2007041494, W02007090752, W02007107243, WO2007120621, US2007265252, US2007265304, W02007128568, WO2007132906, WO2008006257, WO2008009435, W02008018529, WO2008058961, W02008058967 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9, DE 10 2006 053635, DE 10 2006 053637, W02007009655-56, WO2008058628, WO2008058629, W02008058630, WO2008058631 beschrieben, verabreicht.

Bei einer Ausführungsform wird die Verbindung der Formel I in Kombination mit Agonisten des GPBAR1 (G-protein-coupled-bile-acid-receptor-1; TGR5), wie sie z.B. in W02007110237, W02007127505, W02008009407 beschrieben sind, verabreicht.

Bei einer Ausführungsform der Erfindung wird die Verbindung der Formel I in Kombination mit einem polymeren Gallensäureadsorber, wie z.B. Cholestyramin, Colesevelam Hydrochlorid, verabreicht.

Bei einer Ausführungsform der Erfindung wird die Verbindung der Formel I in Kombination mit Colesevelam Hydrochlorid und Metformin oder einem Sulfonylharnstoff oder Insulin verabreicht.

Bei einer Ausführungsform der Erfindung wird die Verbindung der Formel I in Kombination mit einem Phytosterole enthaltenden Kaugummi (Reductol^{™}) verabreicht.

Bei einer Ausführungsform der Erfindung wird die Verbindung der Formel I in Kombination mit einem Inhibitor des mikrosomalen Triglycerid-Transfer-Proteins (MTP-Inhibitor), wie z.B. Implitapide, BMS-201038, R-103757, AS-1552133, SLx-4090, AEGR-733 oder solchen wie in WO2005085226, WO2005121091, W02006010423, W02006113910, WO2007143164, WO2008049806, WO2008049808, beschrieben, verabreicht.

Bei einer weiteren Ausführungsform der Erfindung wird die Verbindung der Formel I in Kombination mit einer Kombinbation eines Cholesterolabsorptionsinhibitors, wie z.B. Ezetimibe, und einem Inhibitor des Triglycerid-Transfer-Proteins (MTP-Inhibitor), wie z.B. Implitapide, wie in W02008030382 beschrieben, verabreicht.

Bei einer Ausführungsform der Erfindung wird die Verbindung der Formel I in Kombination mit einem antihypertriglyceridämischen Wirkstoff, wie z.B. solchen wie sie in W02008032980 beschrieben sind, verabreicht.

Bei einer anderen Ausführungsform der Erfindung wird die Verbindung der Formel I in Kombination mit einem Antagonisten des Somatostatin 5 Rezeptors (SST5 Rezeptor), wie z.B. solchen wie sie in WO2006094682 beschrieben sind, verabreicht.

Bei einer Ausführungsform der Erfindung wird die Verbindung der Formel I in Kombination mit einem ACAT-Inhibitor, wie z.B. Avasimibe, SMP-797 oder KY-382, verabreicht.

Bei einer weiteren Ausführungsform der Erfundung wird die Verbindung der Formel I in Kombination mit einem Inhibitor der Leber-Carnitin Palmitoyltransferase-1 (L-CPT1), wie sie z.B. in WO2007063012, WO2007096251 (ST-3473), W02008015081, US2008103182 beschrieben sind, verabreicht.

Bei einer weiteren Ausführungsform der Erfindung wird die Verbindung der Formel I in Kombination mit einem Modulator der Serin-Palinitoyltransferase (SPT), wie sie z.B. in WO2008031032, WO2008046071 beschrieben sind, verabreicht.

Bei einer Ausführungsform der Erfindung wird die Verbindung der Formel I in Kombination mit einem Squalen Synthetase Inhibitor, wie z.B. BMS-188494, TAK-475 (Lapaquistat Acetat) oder wie in WO2005077907, JP2007022943, W02008003424 beschrieben, verabreicht.

Bei einer Ausführungsform der Erfindung wird die Verbindung der Formel I in Kombination mit ISIS-301012 (Mipomersen), einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738, WO2008020607 beschrieben, verabreicht.

Bei einer anderen Ausführungsform der Erfindung wird die Verbindung der Formel I in Kombination mit einem HDL-Cholesterol-erhöhenden Agens, wie z.B. solchen wie sie in WO2008040651 beschrieben sind, verabreicht.

Bei einer Ausführungsform der Erfindung wird die Verbindung der Formel I in Kombination mit einem ABCA1 Expressionsverstäker, wie sie z.B. in WO2006072393 beschrieben, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit einem Adenosin A1 Rezeptor Agonisten (Adenosin A1 R), wie sie z.B. in EP1258247, EP1375508, W02008028590 beschrieben sind, verabreicht.

Bei einer Ausführungsform der Erfindung wird die Verbindung der Formel I in Kombination mit einem Adenosin A2B Rezeptor Agonisten (Adenosin A2B R) wie z.B. ATL-801 verabreicht.

Bei einer anderen Ausführungsform der Erfindung wird die Verbindung der Formel I in Kombination mit einem Modulator der Adenosin A2A und/oder Adenosin A3 Rezeptoren, wie z.B. in WO2007111954, WO2007121918, WO2007121921, WO2007121923 beschrieben, verabreicht.

Bei einer Ausführungsform der Erfindung wird die Verbindung der Formel I in Kombination mit einem Adenosin A2B Rezeptor Antagonisten (Adenosin A2B R), wie sie in US2007270433, WO2008027585 beschrieben sind, verabreicht.

Bei einer Ausführungsform wird die Verbindung der Formel I in Kombination mit Hemmstoffen der Acetyl-CoA Carboxylase (ACC1 und/oder ACC2) wie z. B. solchen wie in WO199946262, W0200372197, W02003072197, WO2005044814, WO2005108370, JP2006131559, WO2007011809, W02007011811, WO2007013691, WO2007095601-603, WO2007119833 beschrieben, verabreicht.

Bei einer anderen Ausführungsform wird die Verbindung der Formel I in Kombination mit Modulatoren der mikrosomalen Acyl-CoA:Glycerol-3-Phosphat-Acyltransferase 3 (GPAT3, beschrieben in WO2007100789) oder mit Modulatoren der mikrosomalen Acyl-CoA:Glycerol-3-Phosphat-Acyltransferase 4 (GPAT4, beschrieben in W02007100833) verabreicht.

Bei einer weiteren Ausführungsform wird die Verbindung der Formel I in Kombination mit Modulatoren der Xanthin-Oxidoreductase (XOR) verabreicht.

Bei einer anderen Ausführungsform wird die Verbindung der Formel I in Kombination mit Inhibitoren der löslichen Epoxidhydrolase (sEH), wie sie z.B. in WO2008051873, WO2008051875 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
NPY-5 Rezeptorantagonisten wie L-152804 oder die Verbindung "NPY-5-BY" der Firma Banyu oder wie sie z. B. in WO2006001318, W02007103295, WO2007125952, WO2008026563, W02008026564, W02008052769 beschrieben sind;
NPY-4-Rezeptorantagonisten wie sie z. B. in W02007038942 beschrieben sind;
NPY-2-Rezeptorantagonisten wie sie z. B. in W02007038943 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424, WO2006095166, W02008003947 beschrieben sind;
Derivaten des Peptids Obestatin wie sie W02006096847 beschrieben sind;
CB 1 R (Cannabinoid Rezeptor 1) Antagonisten wie z.B. Rimonabant, Surinabant (SR147778), SLV-319 (Ibipinabant), AVE-1625, Taranabant (MK-0364) oder Salze davon, Otenabant (CP-945,598), V-24343 oder solche Verbindungen wie sie in z. B. EP 0656354, WO 00/15609, WO2001/64632-64634, WO 02/076949, WO2005080345, WO2005080328, WO2005080343, WO2005075450, W02005080357, W0200170700, WO2003026647-48, WO200302776, WO2003040107, W02003007887, W02003027069, US6,509,367, WO200132663, WO2003086288, WO2003087037, WO2004048317, WO2004058145, W02003084930, W02003084943, WO2004058744, W02004013120, WO2004029204, W02004035566, WO2004058249, WO2004058255, WO2004058727, WO2004069838, US20040214837, US20040214855, US20040214856, W02004096209, WO2004096763, WO2004096794, WO2005000809, WO2004099157, US20040266845, W02004110453, WO2004108728, W02004000817, WO2005000820, US20050009870, W0200500974, W02004111033-34, W0200411038-39, W02005016286, W02005007111, WO2005007628, US20050054679, W02005027837, WO2005028456, WO2005063761-62, WO2005061509, W02005077897, W02006018662, WO2006047516, W02006060461, WO2006067428, W02006067443, W02006087480, WO2006087476, WO2006100208, WO2006106054, W02006111849, W02006113704, WO2007009705, W02007017124, WO2007017126, W02007018459, W02007018460, WO2007016460, W02007020502, WO2007026215, W02007028849, W02007031720, WO2007031721, W02007036945, WO2007038045, W02007039740, US20070015810, WO2007046548, WO2007047737, W02007057687, WO2007062193, WO2007064272, WO2007079681, W02007084319, WO2007084450, W02007086080, EP1816125, US2007213302, WO2007095513, WO2007096764, US2007254863, WO2007119001, WO2007120454, WO2007121687, WO2007123949, US2007259934, W02007131219, W02007133820, WO2007136571, W02007136607, W02007136571, US7297710, W02007138050, W02007139464, W02007140385, WO2007140439, W02007146761, WO2007148061, WO2007148062, US2007293509, W02008004698, WO2008017381, US2008021031, WO2008024284, WO2008031734, W02008032164, WO2008034032, WO2008035356, WO2008036021, WO2008036022, W02008039023, W02998043544, WO2008044111, WO2008048648, EP1921072-A1, WO2008053341, W02008056377, W02008059207, WO2008059335 beschrieben sind;
Cannabinoid Rezeptor 1/ Cannabinoid Rezeptor 2 (CB1/CB2) modulierende Verbindungen wie z.B. delta-9-Tetrahydrocannabivarin oder solchen wie sie z.B. in W02007001939, WO2007044215, WO2007047737, W02007095513, WO2007096764, W02007112399, W02007112402 beschrieben sind;
Modulatoren der FAAH (fatty acid amide hydrolase) wie sie z.B. in W02007140005, WO2008019357, WO2008021625, WO2008023720, WO2008030532 beschrieben sind;
Vanilloid-1-Rezeptor Modulatoren (Modulatoren des TRPV1), wie sie z.B. in WO2007091948, WO2007129188, WO2007133637, WO2008007780, WO2008010061, W02008007211, W02008010061, WO2008015335, WO2008018827, WO2008024433, WO2008024438, W02008032204, WO2008050199, WO2008059370 beschrieben sind;
Antagonisten oder inverse Agonisten der Opioidrezeptoren, wie sie z.B. in WO2008021849, WO2008021851, WO2008032156 beschrieben sind;
Agonisten des Prostaglandinrezeptors, wie z.B. Bimatoprost oder solchen Verbindungen wie sie in W02007111806 beschrieben sind;
MC4-Rezeptor Agonisten (Melanocortin-4 Rezeptor Agonisten, MC4R Agonisten wie z.B. 1-Amino-1,2,3,4-tetrahydro-naphthalin-2-carbonsäure [2-(3a-benzyl-2-methyl-3-oxo-2,3,3a,4,6,7-hexahydro-pxazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141, MK-0493 oder solche wie sie in WO2005060985, W02005009950, WO2004087159, WO2004078717, WO2004078716, WO2004024720, US20050124652, WO2005051391, W02004112793, WOUS20050222014, US20050176728, US20050164914, US20050124636, US20050130988, US20040167201, W02004005324, WO2004037797, WO2005042516, W02005040109, WO2005030797, US20040224901, WO200501921, WO200509184, WO2005000339, EP1460069, WO2005047253, W02005047251, W02005118573, EP1538159, WO2004072076, WO2004072077, WO2006021655-57, WO2007009894, WO2007015162, WO2007041061, WO2007041052, JP2007131570, EP-1842846, W02007096186, W02007096763, W02007141343, WO2008007930, WO2008017852, W02008039418 beschrieben sind;
Orexin-Rezeptor 1 Antagonisten (OX1R Antagonisten), Orexin-Rezeptor 2 Antagonisten (OX2R Antagonisten) oder gemischte OX1R/OX2R Antagonisten (z.B. 1-(2-Methyl-benzoxazol-6-yl)-3-[1,3]naphthyridin-4-yl-harnstoff Hydrochlorid (SB-334867-A) oder solche, wie sie z. B. in WO200196302, WO200185693, WO2004085403, WO2005075458, WO2006067224, WO2007085718, WO2007088276, WO2007116374;WO2007122591, WO2007126934, WO2007126935, WO2008008517, WO2008008518, WO2008008551, WO2008020405, W02008026149, WO2008038251 beschrieben sind);
Histamin H3 Rezeptor Antagonisten/inverse Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893, US2005171181 (z.B. PF-00389027), W02006107661, WO2007003804, WO2007016496, W02007020213, WO2007049798, W02007055418, W02007057329, WO2007065820, WO2007068620, WO2007068641, W02007075629, WO2007080140, WO2007082840, WO2007088450, WO2007088462, W02007094962, WO2007099423, WO2007100990, WO2007105053, WO2007106349, W02007110364, W02007115938, W02007131907, W02007133561, US2007270440, W02007135111, W02007137955, US2007281923, W02007137968, W02007138431, W02007146122, WO2008005338, WO2008012010, WO200801525 WO2008045371 beschrieben sind);
Histamin H1 / Histamin H3 Modulatoren, wie z. B. Betahistin bzw. seinem Dihydrochlorid;
Modulatoren des Histamin H3 Transporters oder der Histamin H3 / Serotonin Transporter wie sie z.B. in WO2008002816, W02008002817, WO2008002818, WO2008002820 beschrieben sind;
Histamin H4 Modulatoren wie sie z.B. in W02007117399 beschrieben sind;
CRF-Antagonisten (z.B. [2-Methyl-9-(2,4,6-trimethyl-phenyl)-9H-1,3,9-triaza-fluoren-4-yl]-dipropyl-amin (WO 00/66585) oder solche CRF1-Antagonisten, wie sie in W02007105113, WO2007133756, WO2008036541, WO2008036579 beschrieben sind);
CRF BP-Antagonisten (z.B. Urocortin);
Urocortin-Agonisten;
Agonisten des beta-3 Adrenoceptors wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553, W02002038543, WO2002038544, WO2007048840-843 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71 (AMG-071, AMG-076), GW-803430 oder solche Verbindungen, wie sie in WO2005085200, WO2005019240, W02004011438, WO2004012648, W02003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293. WO2006044174, JP2006176443, WO2006018280, W02006018279, W02006118320, WO2006130075, W02007018248, WO2007012661, W02007029847, WO2007024004, WO2007039462, WO2007042660, WO2007042668, W02007042669, US2007093508, US2007093509, WO2007048802, JP2007091649, W02007092416; WO2007093363-366, WO2007114902, W02007114916, WO2007141200, WO2007142217, US2007299062, WO2007146758, WO2007146759, W02008001160, W02008015558, W02008016811, WO2008020799, WO2008022979, W02008038692, WO2008041090, WO2008044632, WO2008047544 beschrieben sind);
CCK-A (CCK-1) Agonisten (wie z.B. {2-[4-(4-Chloro-2,5-dimethoxy-phenyl)-5-(2-cyclohexylethyl)-thiazol-2-ylcarbamoyl]-5,7-dimethyl-indol-1-yl}-essigsäure. Trifluoressigsäuresalz (WO 99/15525) oder SR-146131 (WO 0244150) oder SSR-125180) oder solchen, wie sie in W02005116034, WO2007120655, WO2007120688, W02007120718 beschrieben sind;
Serotonin-Wiederaufnahme-Inhibitoren (z.B. Dexfenfluramine) oder solchen wie sie in W02007148341, WO2008034142 beschrieben sind;
gemischte Serotonin-/Dopamin-Wiederaufnahme-Inhibitoren (z.B. Bupropion) oder solche wie sie in W02008063673 beschrieben sind oder feste Kombinationen von Bupropion mit Naltrexon oder Bupropion mit Zonisamid;
gemischte Wiederaufnahmeinhibitoren wie z.B. DOV-21947;
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
gemischte Dopamin/Norepinephrin/Acetylcholin-Wiederaufnahme-Inhibitoren (z.B. Tesofensine) oder solchen wie sie z.B. in W02006085118 beschrieben sind;
Norepinephrin-Wiederaufnahme-Inhibitoren wie sie z.B. in US2008076724 beschrieben sind;
5-HT2A Rezeptor Antagonisten wie sie z.B. in W02007138343 beschrieben sind;
5-HT2C Rezeptor Agonisten (wie z.B. Lorcaserin Hydrochlorid (APD-356) oder BVT-933 oder solche, wie sie in WO200077010, WO200077001-02, W02005019180, W02003064423, WO200242304, W02005035533, W02005082859, WO2006004937, US2006025601, W02006028961, W02006077025, WO2006103511, WO2007028132, W02007084622, US2007249709; WO2007132841, WO2007140213, W02008007661, W02008007664, WO2008009125, WO2008010073 beschrieben sind);
5-HT6 Rezeptor Modulatoren, wie z.B. E-6837, BVT-74316 oder PRX-07034 oder solche wie sie z.B. in WO2005058858, WO2007054257, WO2007107373, WO2007108569, WO2007108742-744, WO2008003703, WO2008027073, WO2008034815, WO2008054288 beschrieben sind;
Agonisten des Estrogenrezeptors gamma (ERR□ Agonisten), wie sie z.B. in W02007131005, WO2008052709 beschrieben sind;
Sigma-1 Rezeptorantagonisten, wie sie z.B. in WO2007098953, WO2007098961, W02008015266, WO2008055932, W02008055933 beschrieben sind;
Muscarin 3 Rezeptor (M3R) Antagonisten, wie sie z.B. in W02007110782, W02008041184 beschrieben sind;
Bombesin-Rezeptor Agonisten (BRS-3 Agonisten), wie sie z.B. in W02008051404, WO2008051405, WO2008051406 beschrieben sind;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695));
Growth Hormone Secretagogue Receptor Antagonisten (Ghrelin Antagonisten) wie z. B. A-778193 oder solchen, wie sie in WO2005030734, WO2007127457, W02008008286 beschrieben sind;
Growth Hormone Secretagogue Receptor Modulatoren wie z.B. JMV-2959, JMV-3002, JMV-2810, JMV-2951 oder solchen, wie sie in WO2006012577 (z.B. YIL-781 oder YIL-870), W02007079239 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250, W02005072740, JP2005206492, WO2005013907, W02006004200, W02006019020, WO2006064189, WO2006082952, WO2006120125, W02006113919, WO2006134317, WO2007016538, W02007060140, JP2007131584, W02007071966, W02007126957, WO2007137103, WO2007137107, W02007138304, W02007138311, WO2007141502, WO2007141517, W02007141538, W02007141545, WO2007144571, WO2008011130, W02008011131, WO2008039007, WO2008048991 beschrieben;
Inhibitoren der Monoacylglycerolacyltransferase (2-Acylglycerol-O-Acyltransferase; MGAT) wie sie z.B. in W02008038768 beschrieben sind;
Inhibitoren der Fettsäuresynthase (FAS) wie z.B. C75 oder solchen, wie in W02004005277, WO2008006113 beschrieben;
Inhibitoren der Stearoyl-CoA delta9 Desaturase (SCD1) wie sie z.B. in WO2007009236, W02007044085, WO2007046867, WO2007046868, W020070501124, W02007056846, W02007071023, W02007130075, WO2007134457, WO2007136746, W02007143597, W02007143823, WO2007143824, W02008003753, WO2008017161, WO2008024390, W02008029266, W02008036715, W02008043087, W02008044767, W02008046226, W02008056687 beschrieben sind;
hypoglykämische/hypertriglyceridämische Indolinverbindungen wie sie in WO2008039087 beschrieben sind;
Inhibitoren des "Adipocyte fatty acid-binding protein aP2" wie z.B. BMS-309403; Aktivatoren der Adiponectinsekretion, wie z.B. in WO2006082978 beschrieben;
Promotoren der Adiponectinproduktion, wie z.B. in W02007125946, WO2008038712 beschrieben;
Oxyntomodulin oder Analoga davon;
Oleoyl-Estron
oder Agonisten oder partiellen Agonisten des Schilddrüsenhormonrezeptors (thyroid hormone receptor agonists) wie z. B: KB-2115 (Eprotirome), QRX-431 (Sobetirome) oder DITPA oder solche, wie in W020058279, WO200172692, WO200194293, WO2003084915, WO2004018421, WO2005092316, WO2007003419, WO2007009913, WO2007039125, WO2007110225 W02007110226, W02007128492, WO2007132475, W02007134864, WO2008001959 beschrieben
oder Agonisten des Schilddrüsenhormonrezeptors beta (TR-beta) wie z. B. MB-07811 oder MB-07344, verabreicht.

Bei einer Ausführungsform der Erfindung wird die Verbindung der Formel I in Kombination mit einer Kombination von Epotirome mit Ezetimibe verabreicht.

Bei einer Ausführungsform der Erfindung wird die Verbindung der Formel I in Kombination mit einem Inhibitor der Site-1 Protease (S1P), wie z.B. PF-429242, verabreicht.

Bei einer weiteren Ausführungsform der Erfindung wird die Verbindung der Formel I in Kombination mit einem RNAi Therapeutikum, welches gegen PCSK9 (Proprotein Convertase Subtilisin/Kexin Typ 9) gerichtet ist, verabreicht.

Bei einer Ausführungsform wird die Verbindung der Formel I in Kombination mit Omacor® oder Lovaza™ (Omega-3-Fettsäureester; hochkonzentrierte Ethylester der Eicosapentaensäure und der Docosahexaensäure) verabreicht.

Bei einer Ausführungsform wird die Verbindung der Formel 1 in Kombination mit Lycopin verabreicht.

Bei einer Ausführungsform der Erfindung wird die Verbindung der Formel 1 in Kombination mit einem Antioxidans, wie z.B. OPC-14117, AGI-1067 (Succinobucol), Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer anderen Ausführungsform wird die Verbindung der Formel I in Kombination mit einem Inhibitor der Carboanhydrase Typ 2 (Carbonic anhydrase type 2), wie z.B. solchen, wie in WO2007065948 beschrieben, verabreicht.

Bei einer anderen Ausführungsform wird die Verbindung der Formel I in Kombination mit Topiramat oder einem Derivat davon, wie es in WO2008027557 beschrieben ist, verabreicht.

Bei einer weiteren Ausführungsform wird die Verbindung der Formel I in Kombination mit einer festen Kombination von Topiramat mit Phentermin (Qnexa™) verabreicht.

Bei einer weiteren Ausführungsform wird die Verbindung der Formel I in Kombination mit einer Antisense-Verbindung, z.B. ISIS-377131, verabreicht, welche die Produktion des Glukokortikoidrezeptors inhibiert.

Bei einer anderen Ausführungsform wird die Verbindung der Formel I in Kombination mit einem Aldosteronsynthaseinhibitor und einem Antagonisten des Glucocorticoidrezeptors, einem Cortisolsyntheseinhibitor und/oder einem Antagonisten des Corticotropin-freisetzenden Faktors (corticotropin releasing factor), wie z.B. in EP1886695 beschrieben, verabreicht.

Bei einer Ausführungsform wird die Verbindung der Formel I in Kombination mit einem Agonisten des RUP3 Rezeptors, wie z. B. in WO2007035355, WO2008005576 beschrieben, verabreicht.

Bei einer anderen Ausführungsform wird die Verbindung der Formel I in Kombination mit einem Aktivator des Gens, welches für die Ataxia Telangiectasia Mutated (ATM) Proteinkinase kodiert, wie z. B. Chloroquin, verabreicht.

Bei einer Ausführungsform wird die Verbindung der Formel I in Kombination mit einem Tau-Protein-Kinase-1-Inhibitor (TPK1 Inhibitor), wie z. B. in W02007119463 beschrieben, verabreicht.

Bei einer Ausführungsform wird die Verbindung der Formel I in Kombination mit einem "c-Jun N-terminal kinase" Inhibitor (JNK-Inhibitor), wie z. B. in WO2007125405, W02008028860 beschrieben, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Modulatoren des Glukokortikoidrezeptors (GR), wie z.B. KB-3305 oder solchen Verbindungen wie sie z. B. in W02005090336, WO2006071609, WO2006135826, W02007105766 beschrieben sind, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1 (einer NAD⁺-abhängigen Proteindeacetylase); dieser Wirkstoff kann z.B. Resveratrol in geeigneten Formulierungen sein, oder solche Verbindungen wie sie in W02007019416 (z.B. SRT-1720) genannt sind.

Bei einer Ausführungsform der Erfindung ist der weitere Wirkstoff DM-71 (N-Acetyl-L-Cystein mit Bethanechol).

Bei einer Ausführungsform wird die Verbindung der Formel I in Kombination mit antihypercholesterolemisch wirkenden Verbindungen, wie sie z.B. in WO2007107587, WO2007111994 beschrieben sind, verabreicht.

Bei einer anderen Ausführungsform wird die Verbindung der Formel I in Kombination mit einem cyclischen Peptidagonisten des VPAC2 Rezeptors, wie sie z.B. in WO2007101146, WO2007133828 beschrieben sind, verabreicht.

Bei einer weiteren Ausführungsform wird die Verbindung der Formel I in Kombination mit einem Agonisten des Endothelinrezeptors, wie sie z.B. in W02007112069 beschrieben sind, verabreicht.

Bei einer weiteren Ausführungsform wird die Verbindung der Formel I in Kombination mit AKP-020 (Bis(ethylmaltolato)oxovanadium-IV) verabreicht.

Bei einer anderen Ausführungsform wird die Verbindung der Formel I in Kombination mit gewebe-selektiven Androgenrezeptor Modulatoren ("tissue-selective androgen receptor modulators"; SARM), wie sie z.B. in WO2007099200, W02007137874 beschrieben sind, verabreicht.

Bei einer weiteren Ausführungsform wird die Verbindung der Formel I in Kombination mit einem AGE (advanced glycation endproduct) Inhibitor, wie sie z.B. in JP2008024673 beschrieben sind, verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer anderen Ausführungsform der Erfindung ist der weitere Wirkstoff Metreleptin (rekombinantes Methionyl-Leptin) kombiniert mit Pramlintide.

Bei einer weiteren Ausführungsform der Erfindung ist der weitere Wirkstoff das Tetrapeptid ISF-402.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin oder solche Derivate wie sie in W02008034142 beschrieben sind.

Bei einer Ausführungsform ist der weitere Wirkstoff Mazindol oder Phentermin.

Bei einer weiteren Ausführungsform ist der weitere Wirkstoff Geniposidinsäure (geniposidic acid; WO2007100104) oder Derivate davon (JP2008106008).

Bei einer Ausführungsform ist der weitere Wirkstoff ein nasal verabreichter Calciumkanalblocker wie z.B. Diltiazem oder solche, wie sie in US 7,138,107 beschrieben sind.

Bei einer Ausführungsform ist der weitere Wirkstoff ein Inhibitor des Natrium-Calcium-Ionen-Austausches wie z.B. solche, wie sie in WO2008028958 beschrieben sind.

Bei einer weiteren Ausführungsform ist der weitere Wirkstoff ein Blocker von Calciumkanälen wie z.B. des CaV3.2 wie sie in WO2008033431, W02008033447, WO2008033356, WO2008033460, W02008033464, WO2008033465, W02008033468 beschrieben sind.

Bei einer Ausführungsform ist der weitere Wirkstoff ein Blocker des "T-type calcium channel" wie sie z.B. in WO2008033431 beschrieben sind.

Bei einer Ausführungsform ist der weitere Wirkstoff ein Inhibitor des KCNQ-Kaliumkanal-2 bzw. -3 wie z.B. solche, wie sie in US2008027049, US2008027090 beschrieben sind.

Bei einer Ausführungsform ist der weitere Wirkstoff ein Inhibitor des Kalium Kv1.3 Ionenkanals wie z.B. solchen, wie sie in WO2008040057, WO2008040058, WO2008046065 beschrieben sind.

Bei einer anderen Ausführungsform ist der weitere Wirkstoff ein Modulator des MCP-1 Rezeptors (monocyte chemoattractant protein-1 (MCP-1)) wie z.B. solche, wie sie in WO2008014360, WO2008014381 beschrieben sind.

Bei einer Ausführungsform ist der weitere Wirkstoff ein Modulator des Somatostatinrezeptors 5 (SSTR5) wie z.B. solche, wie sie in W02008019967, US2008064697 beschrieben sind.

Bei einer Ausführungsform ist der weitere Wirkstoff ein Modulator des Somatostatinrezeptors 2 (SSTR2) wie z.B. solche, wie sie in WO2008051272 beschrieben sind.

Bei einer Ausführungsform ist der weitere Wirkstoff ein Erythropoietin-mimetisches Peptid, welches als Erythropoietin (EPO) Rezeptoragonist agiert. Solche Moleküle sind z.B. in WO2008042800 beschrieben.

Bei einer weiteren Ausführungsform ist der weitere Wirkstoff ein Anorektikum/eine hypoglykämische Verbindung wie z.B. solche, wie sie in WO2008035305, WO2008035306, WO2008035686 beschrieben sind.

Bei einer Ausführungsform ist der weitere Wirkstoff ein Induktor der Liponsäuresynthetase wie z.B. solche, wie sie in WO2008036966, WO2008036967 beschrieben sind.

Bei einer Ausführungsform ist der weitere Wirkstoff ein Stimulator der endothelialen Nitric-Oxid-Synthase (eNOS) wie z.B. solche, wie sie in WO2008058641 beschrieben sind.

Bei einer Ausführungsform ist der weitere Wirkstoff ein Modulator des Kohlenhydrat- und/oder Lipidstoffwechsels wie z.B. solche, wie sie in W02008059023, W02008059024, W02008059025, WO2008059026 beschrieben sind.

Bei einer weiteren Ausführungsform ist der weitere Wirkstoff ein Angiotensin II Rezeptorantagonist wie z.B. solche, wie sie in WO2008062905 beschrieben sind.

Bei einer Ausführungsform ist der weitere Wirkstoff ein Agonist des Sphingosin-1-Phosphatrezeptors (S1P) wie z.B. solche, wie sie in WO2008064315 beschrieben sind.

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

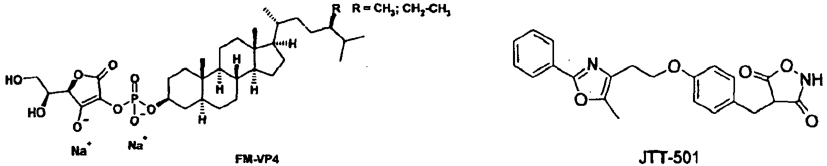

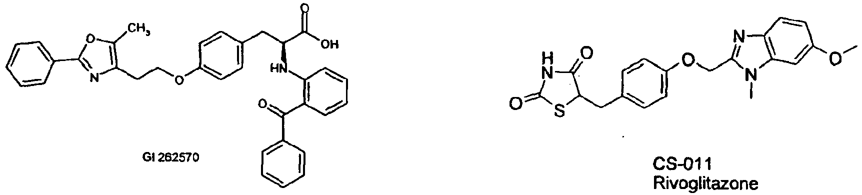

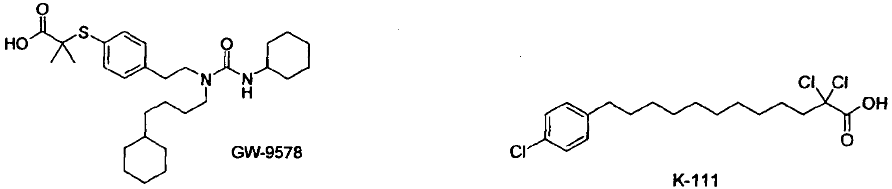

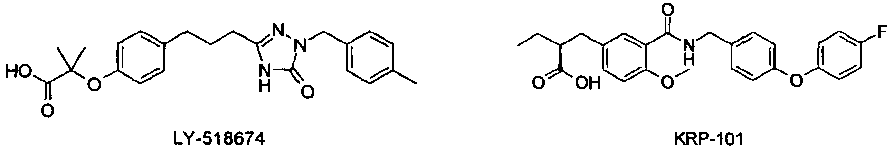

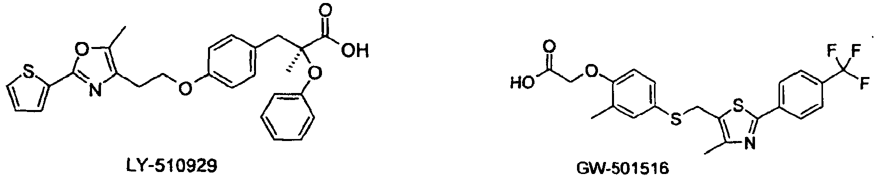

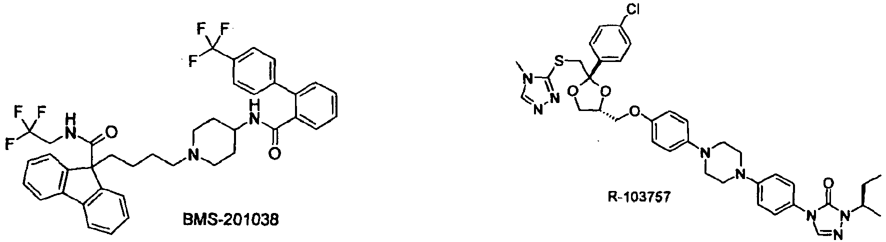

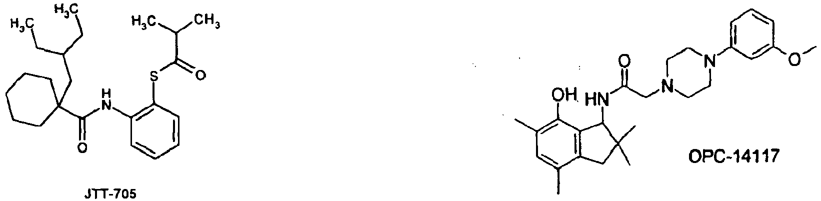

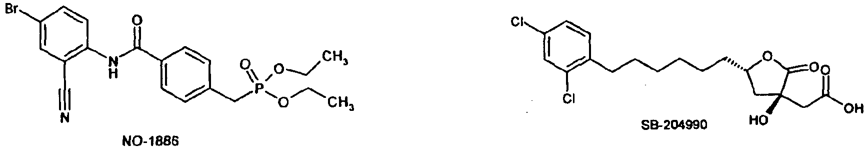

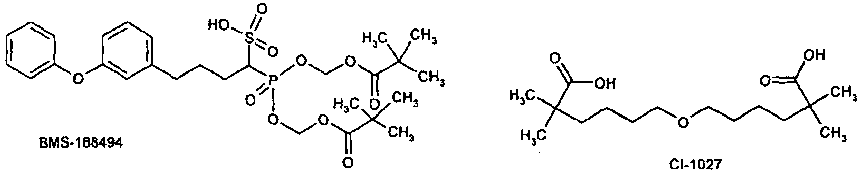

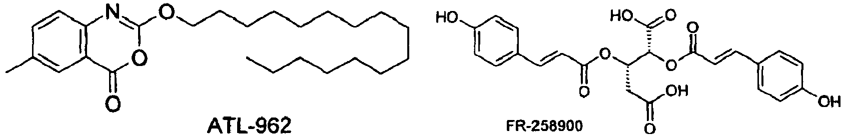

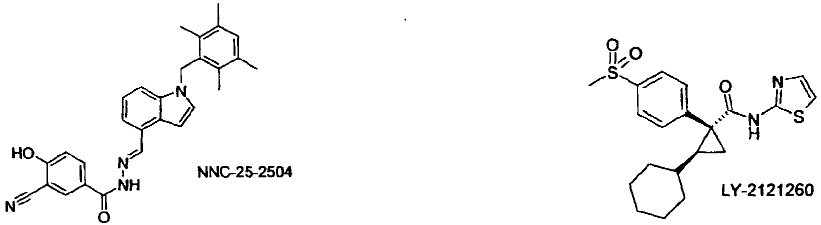

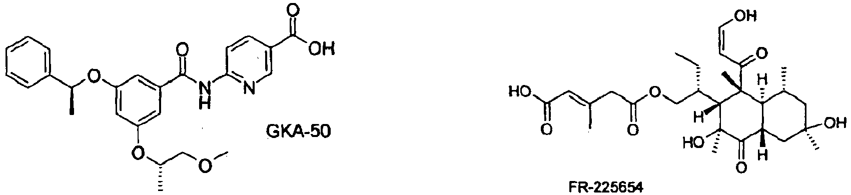

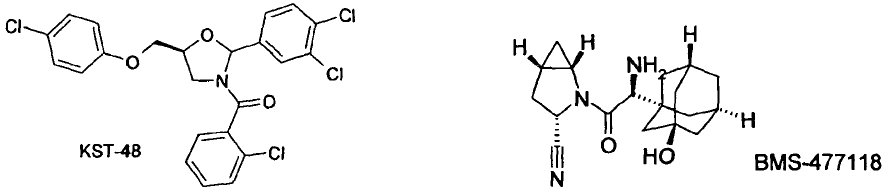

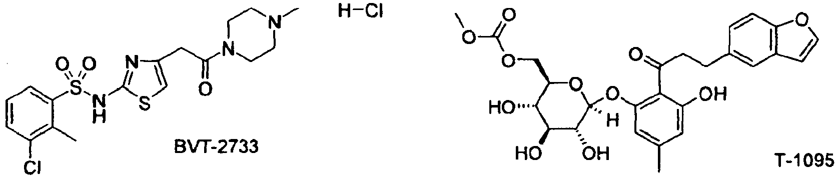

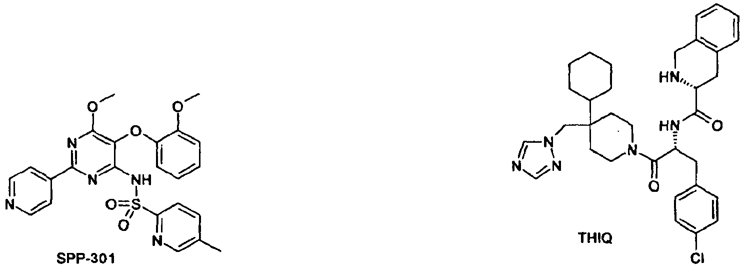

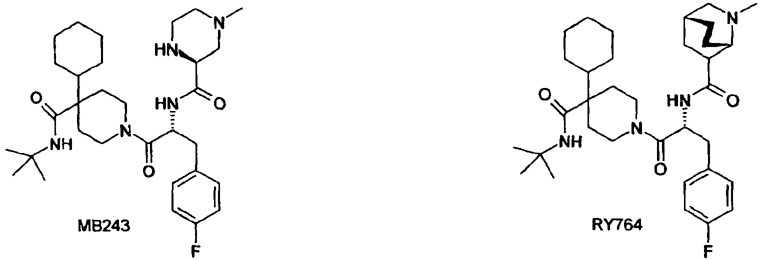

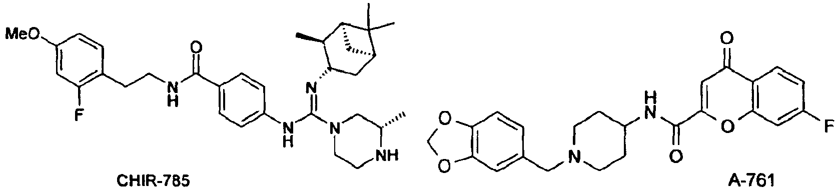

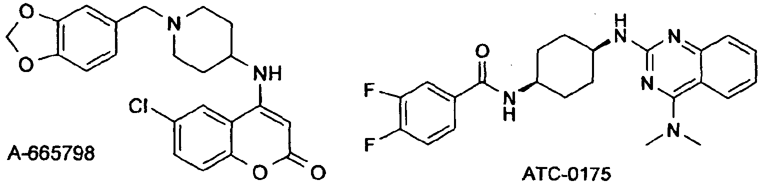

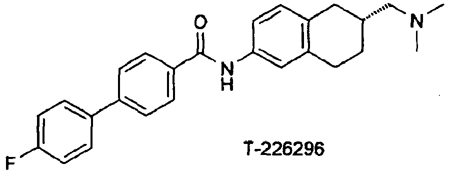

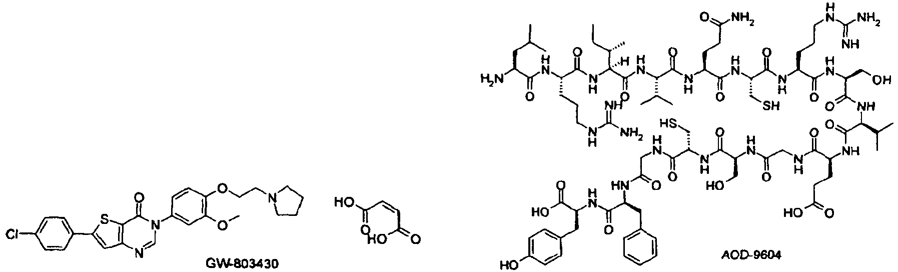

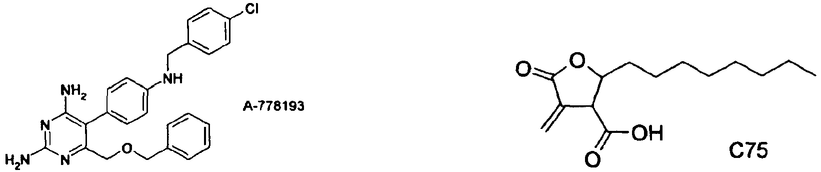

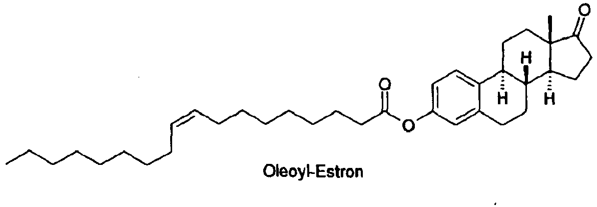

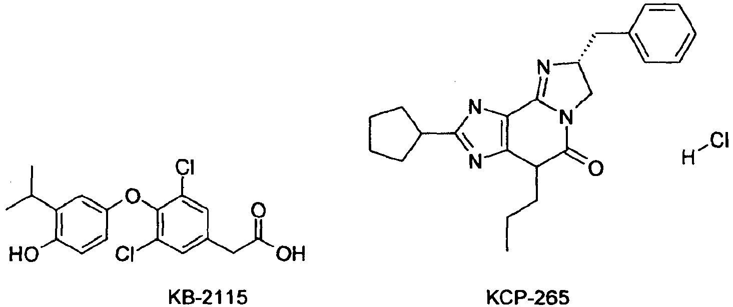

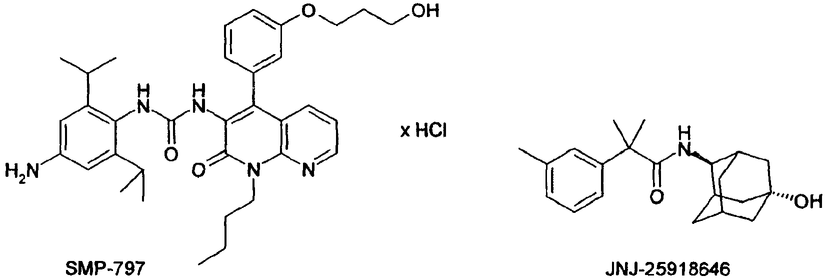

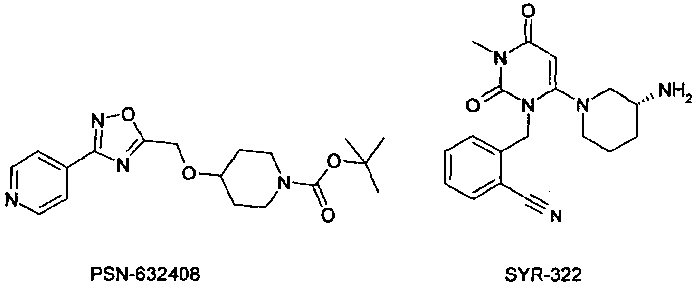

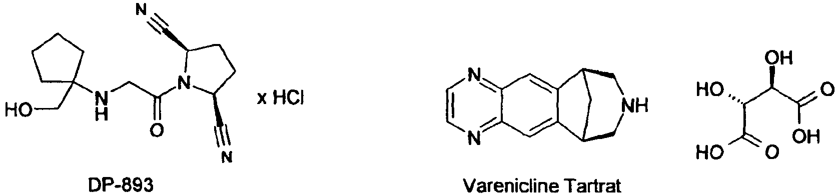

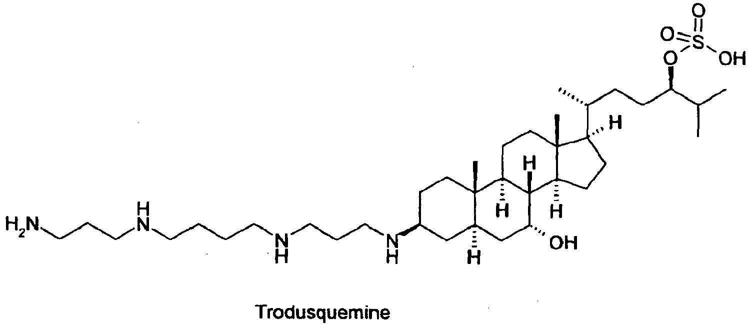

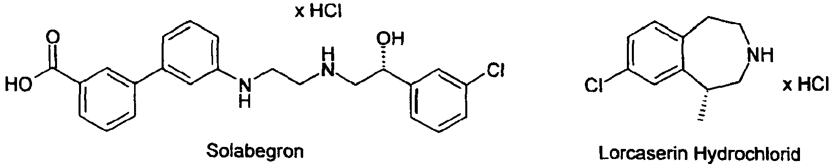

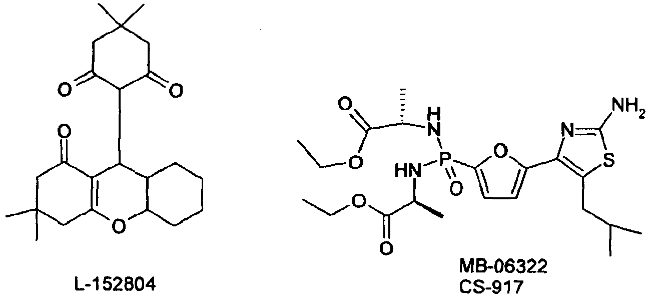

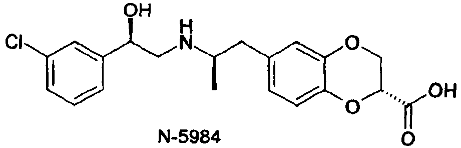

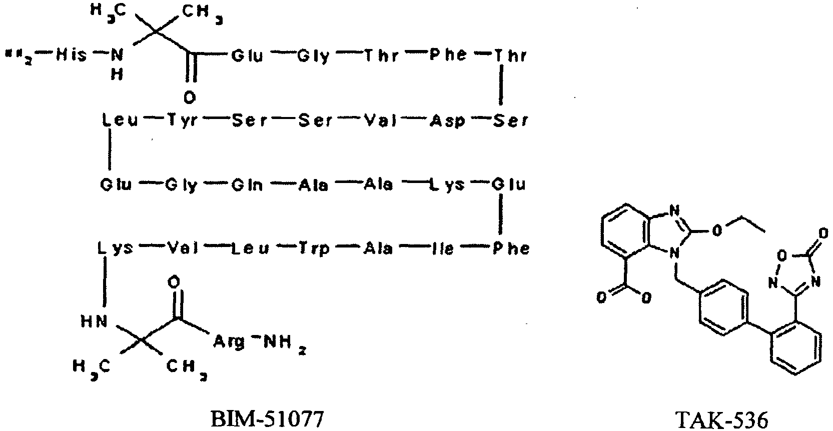

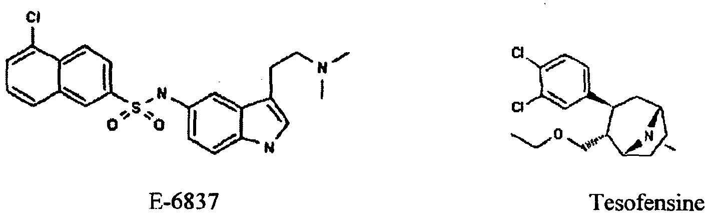

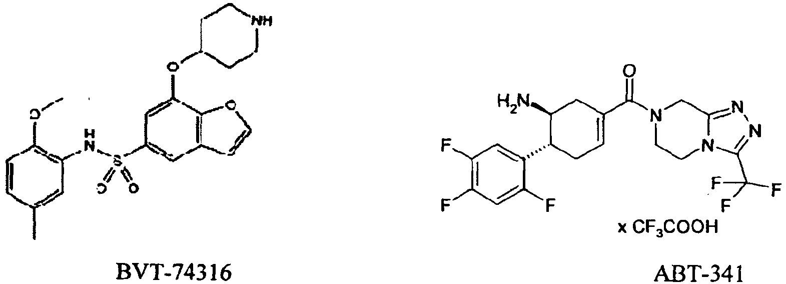

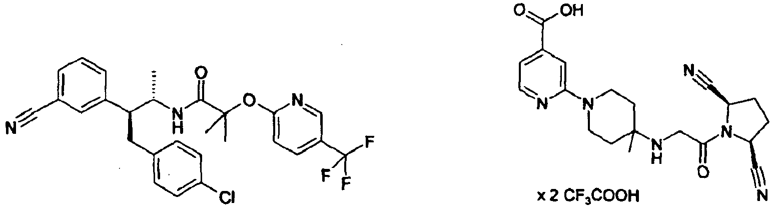

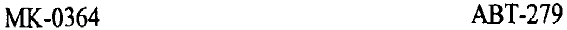

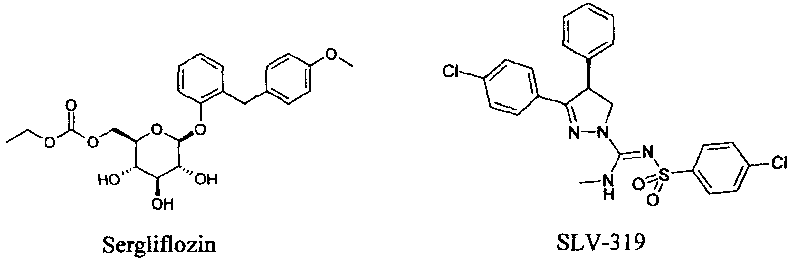

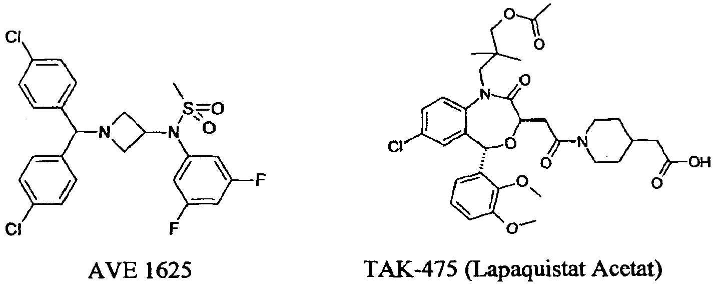

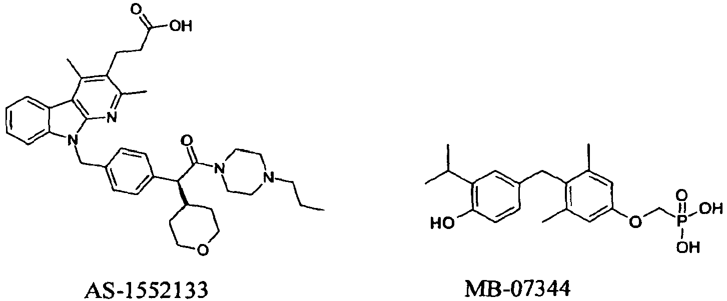

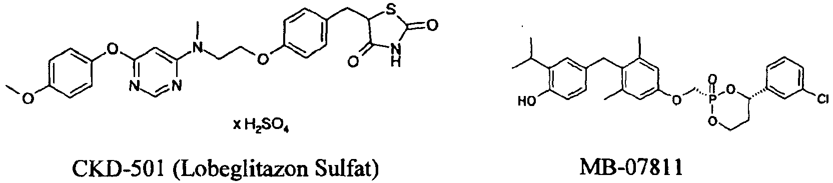

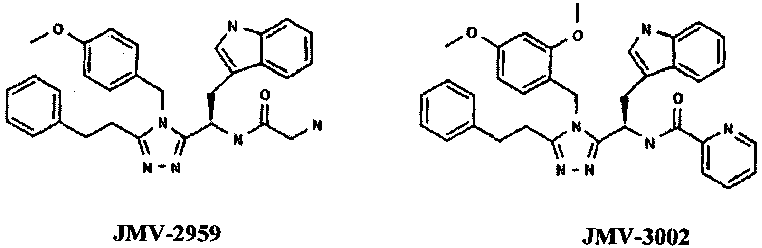

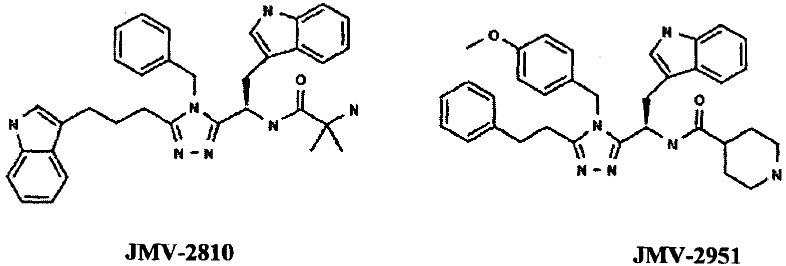

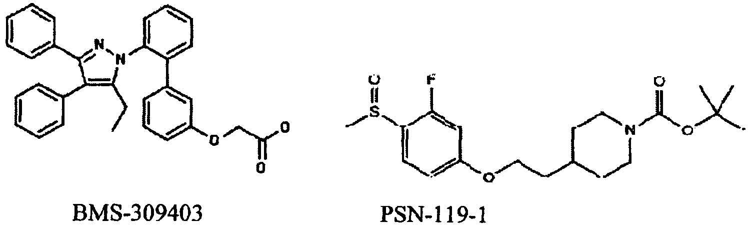

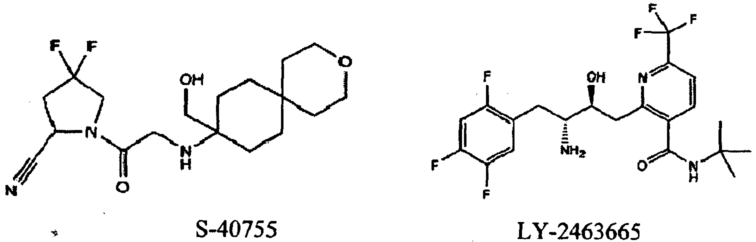

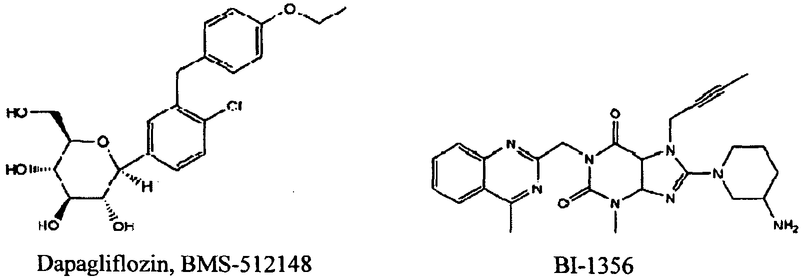

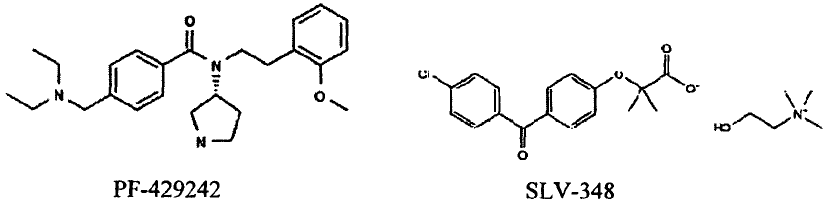

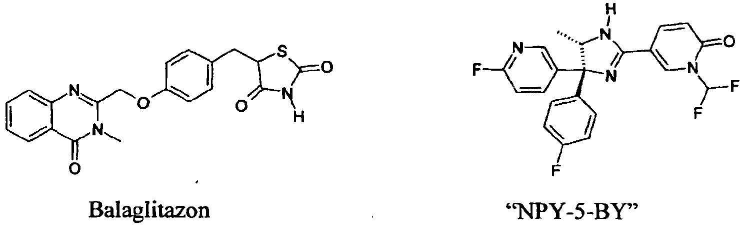

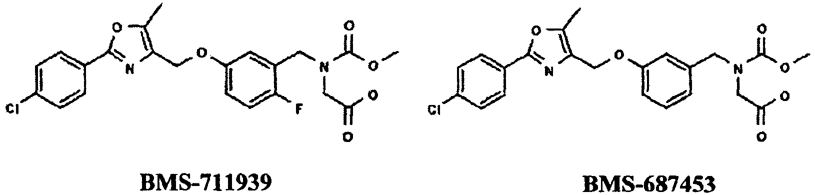

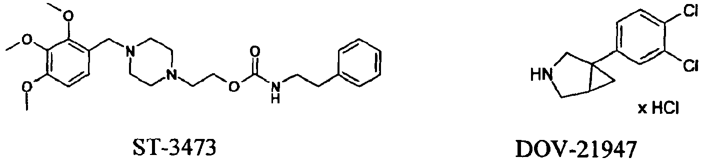

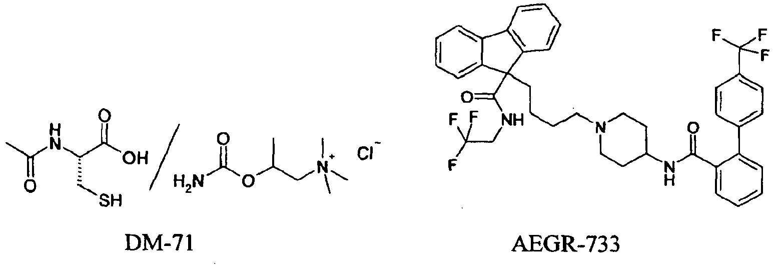

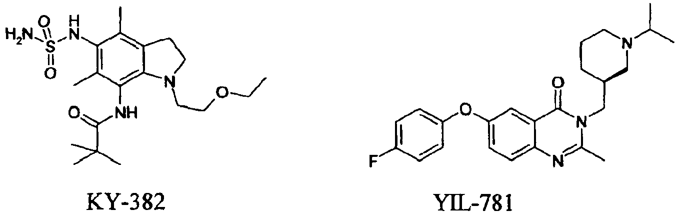

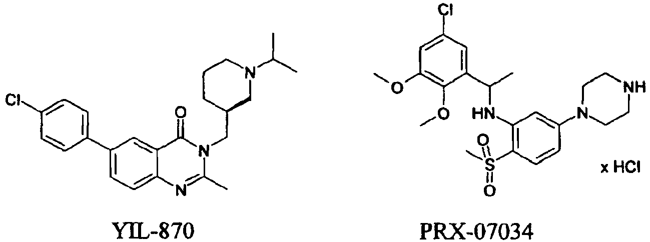

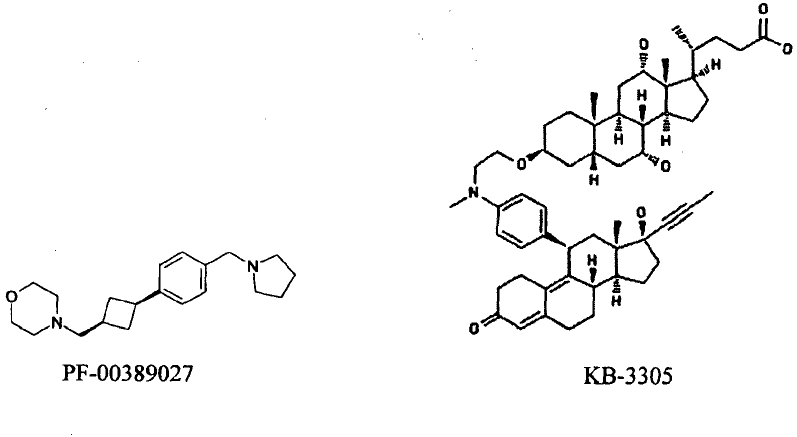

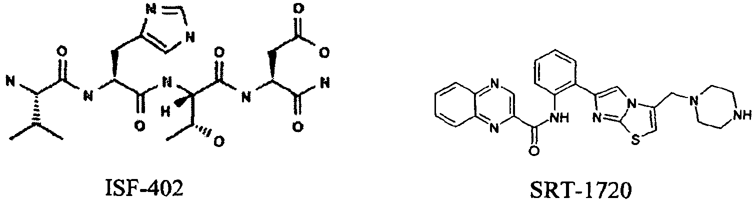

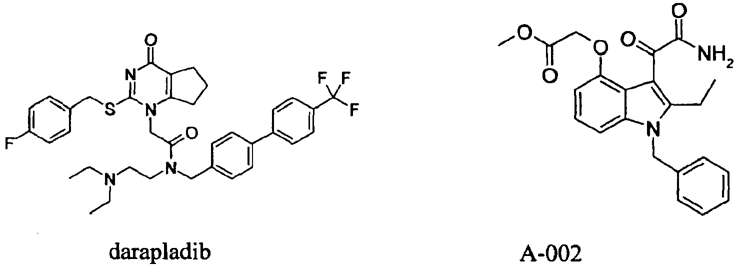

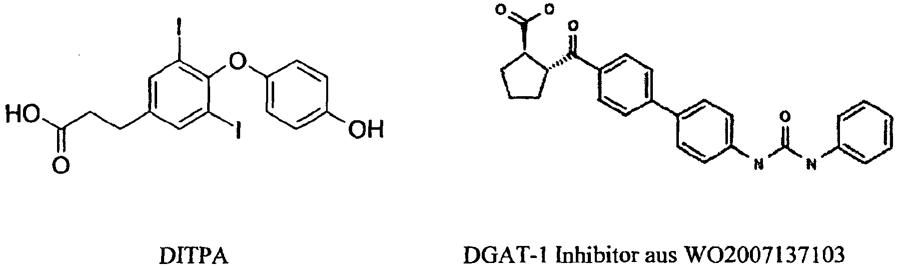

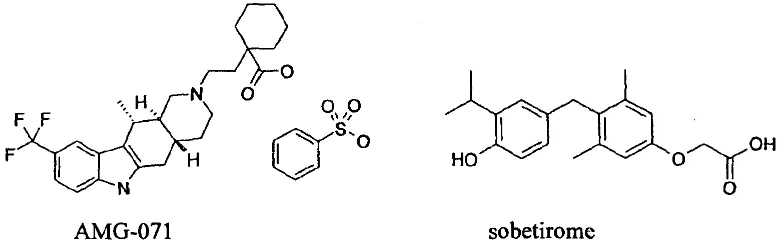

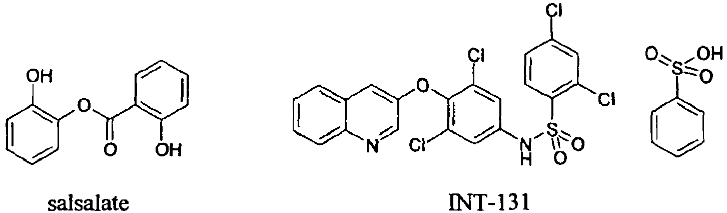

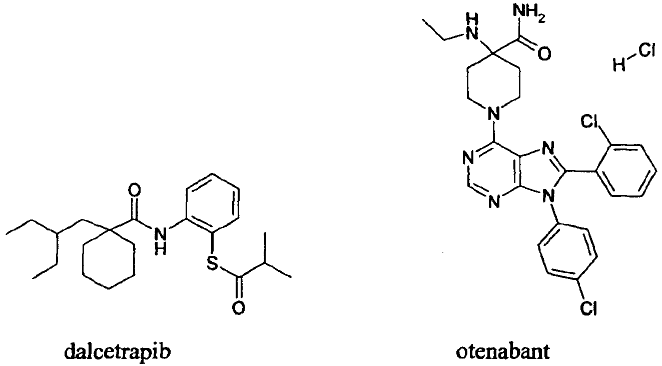

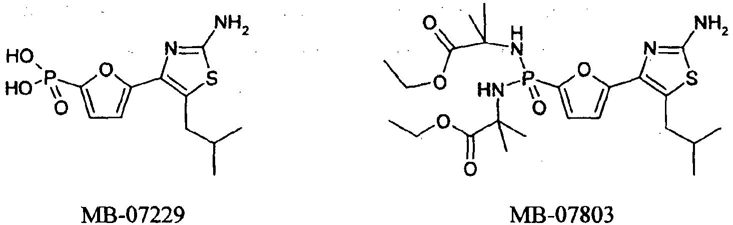

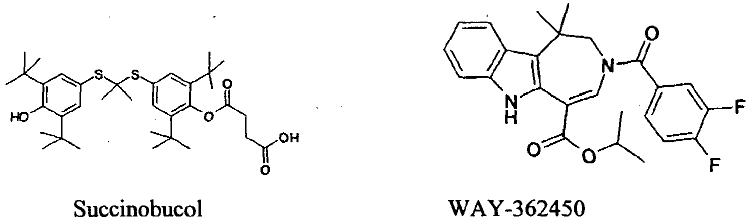

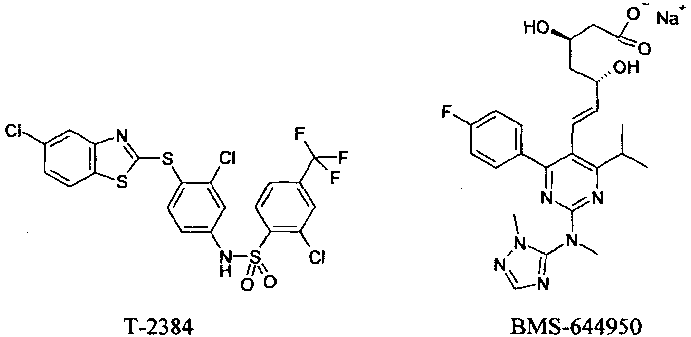

Weiterhin sind folgende Wirkstoffe für Kombinationspräparate geeignet: Alle Antiepileptika, die in der Roten Liste 2007, Kapitel 15 genannt sind; alle Antihypertonika, die in der Roten Liste 2007, Kapitel 17 genannt sind; alle Hypotonika, die in der Roten Liste 2007, Kapitel 19 genannt sind; alle Antikoagulantia, die in der Roten Liste 2007, Kapitel 20 genannt sind;
alle Arteriosklerosemittel, die in der Roten Liste 2007, Kapitel 25 genannt sind;
alle Betarezeptoren-, Calciumkanalblocker und Hemmstoffe des Renin-Angiotensin-Systems, die in der Roten Liste 2007, Kapitel 27 genannt sind;
alle Diuretika und Durchblutungsfördernde Mittel, die in der Roten Liste 2007, Kapitel 36 und 37 genannt sind;
alle Entwöhnungsmittel/mittel zur Behandlung von Suchterkrankungen, die in der Roten Liste 2007, Kapitel 39 genannt sind;
alle Koronarmittel und Magen-Darm-Mittel, die in der Roten Liste 2007, Kapitel 55 und 60 genannt sind;
alle Migränemittel, Neuropathiepräparate und Parkinsonmittel, die in der Roten Liste 2007, Kapitel 61, 66 und 70 genannt sind.

Die Erfindung betrifft weiterhin Verfahren zur Darstellung der Verbindung der Formel I.

### Verfahren A:

Die Verbindung der Formel II 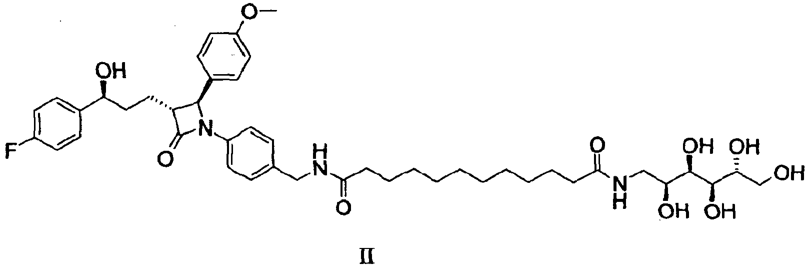 wird durch Chromatographie gereinigt, zum Beispiel an RP 18 Kieselgel mit einem geeigneten Laufmittelsystem z.B. aus Wasser, Acetonitril und Trifluoressigsäure, und anschließend durch Behandlung mit Wasser in das kristalline Hydrat der Formel I übergeführt.

### Verfahren B:

Die Verbindung der Formel II wird in einem organischen Lösungsmittel gelöst. Bevorzugt wird Ethanol als organisches Lösungsmittel verwendet. Zur Erhöhung der Löslichkeit kann Wasser beigefügt werden. Diese Lösung wird zu einer Suspension von Impfkristallen in Wasser zugegeben. Die benötigten Impfkristalle können zum Beispiel nach Verfahren A hergestellt werden. Das Wasser kann auch noch einen Anteil eines organischen Lösungsmittels enthalten.

Der Wassergehalt des Hydrats kann nach dem Trocknen unter Vakuum bei erhöhten Temperaturen auch deutlich unter dem für ein Monohydrat (n=1) berechneten Wert von 2,2 % liegen. Setzt man die Verbindung der Umgebunsluft aus (zum Beispiel mit einer relativen Luftfeuchte von 40 - 60%) nähert sich der Wassergehalt dem theoretischen Wert wieder an.

Nachfolgend wird die Herstellung einiger Beispiele detailliert beschrieben, die übrigen Hydrate der Verbindung der Formel I wurden analog erhalten.

### Experimenteller Teil

### Beispiel 1

Die amorphe Verbindung der Formel II (HPLC-Reinheit: 95,8 %, Wassergehalt: 0,3 %) wird durch Chromatographie gereinigt:
Stationäre Phase: Kromasil C-18, 7 µm
Säulenvolumen: 1,7 L
Säulenlänge: 22 cm
Mobile Phasen:
   A: Wasser / Acetonitril = 9 / (Vol/Vol) versetzt mit 0,1 Vol-% Trifluoressigsäure
   B: Wasser / Acetonitril = 1/9 (Vol/Vol) versetzt mit 0,1 Vol-% Trifluoressigsäure Flußrate: 200 mL/min
Gradiend:
   t= 0 min; 20 % mobile Phase B
   t= 90 min, 47 % mobile Phase B

Substanzaufgabe: 8 g Verbindung II werden in 770 mL mobile Phase B gelöst und mit 3500 ml mobile Phase A verdünnt. Die Lösung wird filtiert und einer Flußrate von 200 mL/min auf die Säule gegeben. Anschließend wird mit dem oben angegebenen Gradienden eluiert.

Fraktionen mit einer Reinheit > 99% werden vereinigt. Am Rotationsverdampfer wird bei einer Badtemperatur < 40 °C das Acetonitril abdestilliert und der Rückstand gefriergetrocknet.

Aus 80 g konnten 60 g der Verbindung der Formel II mit einer HPLC-Reinheit >99 % erhalten werden. Das amorphe Produkt enthielt 0.38 % Wasser und 3,1 % Trifluoressigsäure.

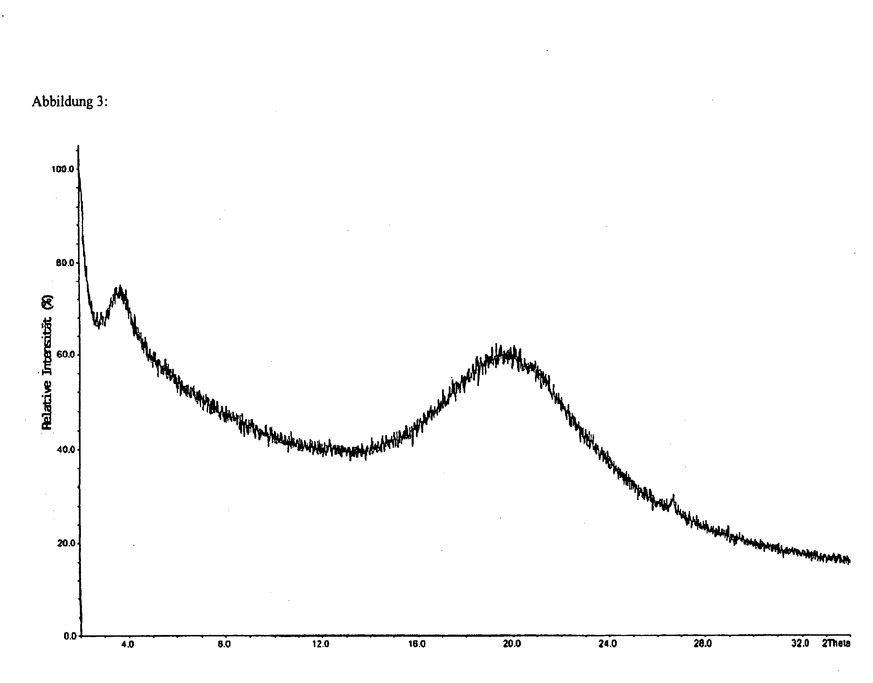

Die Verbindung der Formel II zeigt das in Abbildung 3 wiedergegebene XRPD.

58 g der Verbindung der Formel II mit einer HPLC-Reinheit >99 % in 1 L 2 %-iger Natriumhydrogencarbonatlösung wurden für eine Stunde bei 20 - 25 °C und dann 30 Minuten im Eisbad gerührt. Das Produkt wird abfiltriert und noch insgesamt zweimal wie oben beschrieben mit 1 L Wasser ausgerührt. Dann wird das Produkt im Vakuum bei 50 °C getrocknet. Man erhält 55,5 g der kristallinen Verbindung der Formel I mit einem Wassergehalt von 1,9 %, was n gleich 0,86 entspricht. Aufgrund natürlicher Abweichungen in den Proben oder in der Meßmethode (nach Karl Fischer) können die Werte des Wassergehalts mit einer Genauigkeit von +/- 0.1 % angegeben werden.

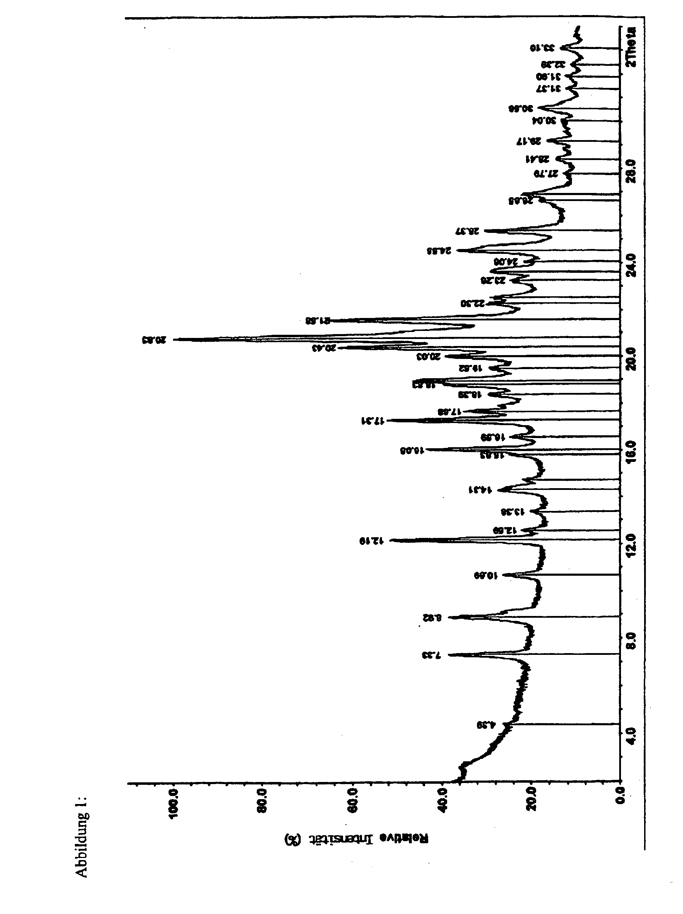

Die erhaltene Verbindung der Formel I zeigt das in Abbildung 1 wiedergegebene XRPD. Die wichtigsten 2 Theta Werte sind in Tabelle 1 zusammengefasst.

**Tabelle 1:**

| 2 Theta (+/- 0,2 Grad 2 Theta) |
|---|
| 4.39 |
| 7.33 |
| 8.92 |
| 10.69 |
| 12.19 |
| 12.59 |
| 13.38 |
| 14.31 |
| 15.83 |
| 16.05 |
| 16.59 |
| 17.31 |
| 17.68 |
| 18.39 |
| 18.83 |
| 19.52 |
| 20.03 |
| 20.43 |
| 20.83 |
| 21.58 |
| 22.30 |
| 23.26 |
| 24.06 |
| 24.55 |
| 25.37 |
| 26.65 |
| 27.79 |
| 28.41 |
| 29.17 |
| 30.04 |
| 30.56 |
| 31.37 |
| 31.90 |
| 32.39 |
| 33.10 |

Aufgrund natürlicher Abweichungen in den Proben oder in der Meßmethode können die 2 Theata Werte der Peaks mit einer Genauigkeit von +/- 0.2 Grad Theta angegeben werden.

### Beispiel 2:

100 g der amorphen Verbindung II (HPLC-Reinheit: 95,8 %, Wassergehalt: 0,3 %) werden in 400 mL wässrigem Ethanol (vergällt mit Methylethylketon, 80 % (Vol/Vol)) durch Erwärmen auf 40 °C gelöst. Man läßt die Lösung auf 20 -25 °C abkühlen.

2 g der kristallinen Verbindung I aus Beispiel 1 werden 20 - 25°C durch Rühren in 10 L destilliertem Wasser suspendiert. Zu dieser Suspension gibt man innerhalb von 70 Minuten die Lösung von Verbindung II in 80 % Ethanol. Das Zugabegefäß wird anschließend noch mit 40 ml 80 % Ethanol nachgespült. Es bildet sich ein weißer Niederschlag. Anschließend rührt man noch 20 h bei 20 - 25 °C. Der Niederschlag wird abgesaugt und portionsweise mit insgesamt 5 L destilliertem Wasser gewaschen. Nach dem Trocknen bei 40 °C im Vakuum erhält man 88.3 g des kristallinen Hydrats der Formel I (Wassergehalt: 2,9 %, was n gleich 1,3 entspricht).

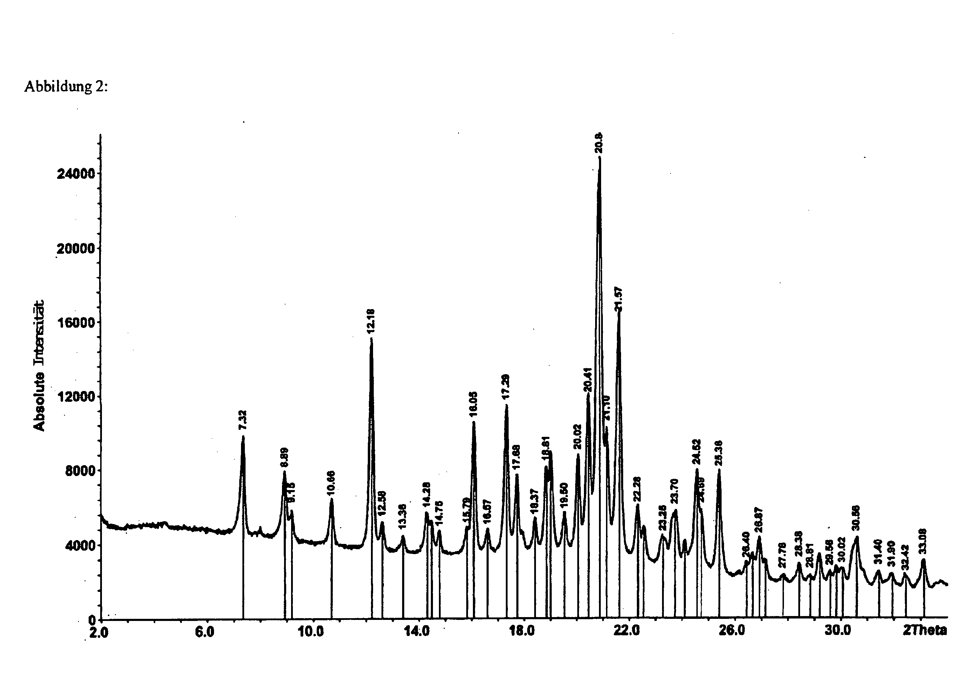

Beispiel 2 zeigt das in Abbildung 2 wiedergegebene XRPD

### Beispiel 3:

25 g der amorphen Verbindung II (HPLC-Reinheit: 95,8 %, Wassergehalt: 0,3 %) werden in 100 mL wässrigem Ethanol (vergällt mit Methylethylketon, 80 % (Vol/Vol)) durch Erwärmen auf 40 °C gelöst. Man läßt die Lösung auf 20 - 25 °C abkühlen.

500 mg der kristallinen Verbindung I aus Beispiel 1 werden bei 20 - 25°C durch Rühren in 500 mL destilliertem Wasser suspendiert Zu dieser Suspension gibt man innerhalb von 60 Minuten die Lösung von Verbindung II in 80 % Ethanol. Das Zugabegefäß wird anschließend noch mit 10 ml 80 % Ethanol nachgespült. Es bildet sich ein weißer Niederschlag. Anschließend rührt man noch 20 h bei 20 - 25 °C. Der Niederschlag wird abgesaugt und mit destilliertem Wasser gewaschen. Nach dem Trocknen bei 45 °C im Vakuum erhält man 24.7 g des kristallinen Hydrats. Das XRPD zeigt Peaks bei gleichen 2Theta Werten wie Beispiel 2.

### Beispiel 4:

10 g der amorphen Verbindung II (HPLC-Reinbeit: 95,8 %, Wassergehalt: 0,3 %) werden in 40 mL wässrigem Ethanol (vergällt mit Methylethyllceton, 80 % (Vol/Vol)) durch Erwärmen auf 40 °C gelöst. Man läßt die Lösung auf 20 -25 °C abkühlen.

0.2 g der kristallinen Verbindung der Formel I aus Bespiel 1 werden bei 20 - 25°C durch Rühren in 1000 mL destilliertem Wasser suspendiert Zu dieser Suspension gibt man innerhalb von 10 Minuten die Lösung von Verbindung II in 80 % Ethanol. Es bildet sich ein weißer Niederschlag, der abgesaugt und erneut durch Rühren in 11 Wasser suspendiert wird. Anschließend rührt man noch 1 h bei 20 - 25 °C. Der Niederschlag wird abgesaugt und mit 20 ml destilliertem Wasser gewaschen. Nach dem Trocknen bei 50 °C im Vakuum erhält man 7.87 g des kristallinen Hydrats der Formel I. Das XRPD zeigt Peaks bei gleichen 2Theta-Werten wie Beispiel 2.

### Beispiel 5:

12 g der amorphen Verbindung II (IPLC-Reinheit: 97,1 %) werden in 35,3 g Ethanol (vergällt mit Methylethylketon) und 9,75 g destiliertem Wasser zum Sieden erhitzt. 2,3 g Flüssigkeit werden abdestilliert. Man läßt die Lösung auf 20 -25 °C abkühlen.

0.12 g der kristallinen Verbindung der Formel I werden bei 20 - 25°C durch Rühren in 414 mL destilliertem Wasser suspendiert. Zu dieser Suspension gibt man innerhalb von 45 Minuten die Lösung von Verbindung II in Ethanol/Wasser. Das Zulaufgefäß wird mit einer Mischung aus 9,75 g Ethanol und 2.25 g Wasser nachgespült. Dann wird 18 h nachgerührt. Der Niederschlag wird abgesaugt und mit 60 ml destilliertem Wasser gewaschen. Nach dem Trocknen bei 40 °C im Vakuum erhält man 11,7 g des kristallinen Hydrats der Formel I (Wassergehalt: 2,3 %, was n = 1,05 entspricht). Das XRPD zeigt Peaks bei gleichen 2Theta-Werten wie Beispiel 2.

### Beispiel 6:

Das im Beispiel 2 erhaltene Hydrat wurde im Vakuum bei 50 °C getrocknet, bis ein Wassergehalt von 0,8 % erreicht wurde. Das kristalline Produkt wurde bei 23 °C/ 60 % relative Luftfeuchtigkeit gelagert.

| Lagerzeit (h) | Wassergehalt (%) | n |
|---|---|---|
| 0,5 | 1,1 | 0,5 |
| 2 | 2,4 | 1,09 |
| 4 | 2,5 | 1,14 |
| 6 | 2,4 | 1,09 |
| 24 | 2,5 | 1,14 |

Das XRPD nach 24 h zeigt Peaks bei gleichen 2Theta-Werten wie Beispiel 2.

### Test der pharmakologischen Wirkung:

Die erfindungsgemäße Verbindung der Hydrate der Formel I wurde mit der nachfolgend beschriebenen Methode auf ihre Wirkung geprüft:

### Beeinflussung der Cholesterolabsorption + ³H- Taurocholsäureausscheidung anhand der fäkalen Ausscheidungan der Maus. Ratte oder Hamster

NMRI- Mäuse, Wistar-Ratten, oder Golden Syrian Hamster (in Gruppen von n=4-6) werden unter Standarddiät (Altromin, Lage (Lippe)) in Stoffwechselkäfigen gehalten. Am Nachmittag vor Gabe der radioaktiven Tracer (¹⁴C-Cholesterol) werden die Tiere nüchtern gesetzt und auf Gitterroste adaptiert.

Zusätzlich werden die Tiere werden 24 Stunden vor der peroralen Applikation der Testmahlzeit (¹⁴C-Cholesterol in Intralipid® 20, Pharmacia-Upjohn) mit ³H-TCA (Taurocholic acid) s.c. gelabelt (z.b. 1 µCi/Maus bis 5 µCi/Ratte)

Cholesterolabsorptionstest: 0,25 ml/Maus Intralipid ® 20 (Pharmacia- Upjohn) ((Spikung mit 0,25 µCi ¹⁴C-Cholesterol in 0,1 mg Cholesterol) werden peroral mit der Schlundsonde verabreicht.

Testsubstanzen werden getrennt in 0,5 %/ (Methylcellulose (Sigma)/5% Solutol (BASF, Ludwigshafen) oder geeignetem Vehikel angesetzt.

Das Applikationsvolumen der Testsubstanz beträgt 0,5 ml /Maus. Die Testsubstanz wird unmittelbar vor der Testmahlzeit (Intralipid mit ¹⁴C-Cholesterol-label)
(Cholesterolabsorptionstest) appliziert.

Der Kot wird über 24 h gesammelt: die fäkale Elimination von ¹⁴C-Cholesterol und ³H Taurocholsäure (TCA) nach 24 Std. wird bestimmt.

Die Lebern werden entnommen, homogenisiert und Aliquots im Oximaten (Model 307, Packard) verbrannt zur Bestimmung der aufgenommenn/resorbierten Menge an ¹⁴C-Cholesterol.

### Auswertung:

### Kotproben:

Gesamtgewicht bestimmen, mit Wasser auf definiertes Volumen auffüllen, dann homogenisieren, Aliquot eintrockenen und im Oximat (Model 307, Packard zur Verbrennung von radioaktiv gelabelten Proben) verbrennen: Die Menge von radioaktiv ³H- H2O und ¹⁴C-CO2 wird hochgerechnet auf die ausgeschiedene Menge an ³H-Taurocholsäure bzw. ¹⁴C-Cholesterol (Dual-Isotopen-Technik ). Die ED₂₀₀-Werte werden als Dosis aus einer Dosiswirkungskurve interpoliert als diejenige Dosen, die die Auscheidung an TCA bzw. Cholesterol verdoppeln, bezogen auf eine zeitgleich behandelte Kontrollgruppe.

### Leberproben:

Die aufgenommene Menge von ¹⁴C-Cholesterols in die Leber wird bezogen auf die applizierte Dosis. Die ED₅₀ Werte werden interpoliert aus einer Dosiswirkungskurve als diejenige Dosis, die die Aufnahme von ¹⁴C- Cholesterol in die Leber halbiert (50%), bezogen auf eine Kontrollgruppe

### Beispiel Nr. 2 zeigt einen ED₂₀₀ - Wert (Fäkale Ausscheidung [mg/Maus]) von 0,01 mg/Maus

Der gemessene ED₂₀₀ -Wert belegt die Aktivität der erfindungsgemäßen Verbindung der Formel I und zeigt, daß die kristallinen Hydrate der Formel 1 eine sehr gute Cholesterin absorptionshemmde Wirkung besitzen.

### Test der Lösegeschwindigkeit:

Die Lösegeschwindigkeit der kristallinen Hydrate der Formel I wurde wie folgt getestet:
Die Lösegeschwindigkeit wird in einer Standard-Apparatur (Paddle) nach US Pharmacopeia (USP) bei 37 °C bestimmt. Die Rührerdrehzahl beträgt 75 Upm.

20 mg der zu untersuchenden Substanz werden in 1000 ml einer 0,1 %igen wässrigen Natriumdodecylsulfat(SDS)-Lösung gegeben. Nach 15, 30, 45 und 60 Minuten werden Proben von 20 ml genommen.

Der gelöste Anteil wird mittels einer Vergleichslösung photometrisch bei 256 nm bestimmt. Die Messung erfolgt in 10 mm Küvetten beispielsweise mit einem Perkin Elmer Lambda 25 Photometer. Die Proben werden direkt nach der Probennahme vermessen. Aus drei Bestimmungen wird jeweils der Mittelwert gebildet

Die Vergleichslöung wird wie folgt hergestellt: 2-3 mg der eingesetzten Charge werden in einem Messkolben mit 2 ml Dimethylformamid gelöst. Anschließend wird mit 0,1 %iger SDS-Lösung auf 100,0 ml aufgefüllt.

Verglichen wurden die in Beispiel 2 eingesetzte amorphe Verbindung der Formel II mit der im gleichen Beispiel 2 erhaltenen kristallinenVerbindung der Formel I. Die Ergebnisse sind in nachfolgender Tabelle zusammengestellt:

| Zeit | Amorphe Verbindung der Formel II | Kristalline Verbindung der Formel I |
|---|---|---|
| [min] | (Freisetzung [%]) | (Freisetzung [%]) |
| 15 | 30,0 | 88,0 |
| 30 | 41,3 | 90,0 |
| 45 | 44,7 | 94,6 |
| 60 | 46,2 | 98,7 |

Die kristallinen Hydrate der Formel I weisen gegenüber der amorphen Verbindung der Formel II aus US 7,205, 290 eine deutlich erhöhte Lösungsgeschwindigkeit auf.

Gegenstand der Erfindung ist damit ein kristallines Hydrat der Formel I, dadurch gekennzeichnet, dass bei der Messung der Lösegeschwindigtkeit in einer Standard-Apparatur nach Paddle USP bei 37°C und einer Rührerdrehzahl von 75 Upm von 20mg des kristallinen Hydrats der Formel I in 1000ml einer 0,1%igen wässrigen Natriumdodecylsulfatlösung nach 15 Minuten bereits mindestens 50% des kristallinen Hydrats der Formel I in Lösung sind.

Bevorzugt ist kristallines Hydrat der Formel I, dadurch gekennzeichnet, dass nach 15 Minuten bereits mindestens 70% des kristallinen Hydrats der Formel I in Lösung sind.

Bevorzugt ist kristallines Hydrat der Formel I, dadurch gekennzeichnet, dass nach 15 Minuten bereits mindestens 85% des kristallinen Hydrats der Formel I in Lösung sind.

Damit Wirkstoffe ihre Wirkung im Körper entfalten können, müssen sie am Wirkort in gelöster Form vorliegen. Dabei sind die Löslichkeit und die Lösegeschwindigkeit in wässrigen Umgebungen zwei wichtige Faktoren. Löslichkeiten von amorphen und kristallinen Verbindungen unterscheiden sich praktisch nicht, da es sich um eine thermodynamische Größe handelt. Dies ist bei der Lösegeschwindigkeit anders, da es sich um eine kinetische Größe handelt. Im Allgemeinen weisen kristalline Verbindungen im Vergleich zu amorphen Verbindungen eine geringere Lösegeschwindigkeit auf, da die kristallinen Verbindungen thermodynamisch stabiler sind.

Daher ist es ein Vorteil, dass das kristalline Hydrat der Formel I gegenüber der amorphen Verbindung II eine deutlich höhere Lösegeschwindigkeit zeigt. Nach bereits 15min ist ein Großteil des Wirkstoffs in Lösung. Dies ist fast die dreifache Menge des amorphen Wirksstoffs. Die Wirkung des kristallinen Hydrats der Formel I wird damit schneller einsetzen als die Wirkung der amorphen Verbindung der Formel II.

Beschreibung der Abbildungen:
Abbildung 1 zeigt das XRPD der Verbindung der Formel I, aus Beispiel 1
Abbildung 2 zeigt das XRPD der Verbindung der Formel I, aus Beispiel 2
Abbildung 3 zeigt das XRPD der Verbindung der Formel II, Edukt der Beispiele 1 bis 4

## Patentansprüche

1. Kristalline Hydrate der Formel I, 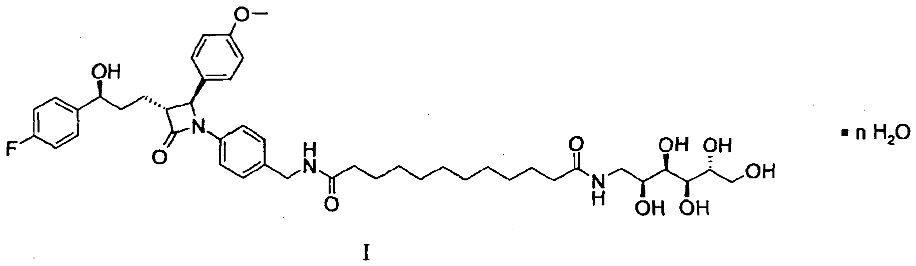 worin n einen Wert von 0,5 bis 1,8 besitzt.

2. Kristalline Hydrate der Formel I, gemäß Anspruch 1, **dadurch gekennzeichnet, dass** n einen Wert von 0,8 bis 1,3 besitzt.

3. Kristallines Hydrat der Formel I, gemäß Anspruch 1 oder 2, **dadurch gekennzeichnet,**
**dass** n einen Wert von 1,0 bis 1,2 besitzt.

4. Kristallines Hydrat der Formel I, gemäß einem oder mehreren der Ansprüche 1 bis 3,
**dadurch gekennzeichnet, dass** das mit CuKα-Strahlung gemessene XRPD einen Hauptpeak von 20.83 Grad 2 Theta ± 0.2 Grad 2 Theta aufweist.

5. Kristallines Hydrat der Formel I, gemäß einem oder mehreren der Ansprüche 1 bis 3,
**dadurch gekennzeichnet, dass** das mit CuKα-Strahlung gemessene XRPD zumindest Peaks von folgenden 2 Theta Werten aufweist: 20.83 und 21.58 ± 0.2 Grad 2 Theta.

6. Kristallines Hydrat der Formel I, gemäß einem oder mehreren der Ansprüche 1 bis 3,
**dadurch gekennzeichnet, dass** das mit CuKα-Strahlung gemessene XRPD zumindest Peaks von folgenden 2 Theta Werten aufweist:
12.19, 20.83 und 21.58 ± 0.2 Grad 2 Theta.

7. Kristallines Hydrat der Formel I, gemäß einem oder mehreren der Ansprüche 1 bis 4, **dadurch gekennzeichnet, dass** das mit CuKα-Strahlung gemessene XRPD zumindest Peaks von folgenden 2 Theta Werten aufweist:
20.43, 20.83, 21.58 ± 0.2 Grad 2 Theta.

8. Kristallines Hydrat der Formel I, gemäß Anspruch 7, **dadurch gekennzeichnet, dass** das mit CuKα-Strahlung gemessene XRPD zumindest Peaks von folgenden 2 Theta Werten aufweist:
12.19, 17.31, 20.43, 20.83, 21.58 ± 0.2 Grad 2 Theta.

9. Kristallines Hydrat der Formel I, gemäß einem oder mehreren der Ansprüche 1 bis 3, **dadurch gekennzeichnet, dass** das mit CuKα-Strahlung gemessene XRPD zumindest Peaks von folgenden 2 Theta Werten aufweist:
7.33, 12.19, 16.05, 17.31,20.83,21.58 ±0.2 Grad 2 Theta.

10. Kristallines Hydrat der Formel I, gemäß Anspruch 8, **dadurch gekennzeichnet, dass** das mit CuKα-Strahlung gemessene XRPD zumindest Peaks von folgenden 2 Theta Werten aufweist:
7.33, 8.92, 12.19, 16.05, 17.31, 17.68, 18.83, 20.43, 20.83, 21.58, 24.55, 25.37 ± 0.2 Grad 2 Theta.

11. Kristallines Hydrat der Formel I, gemäß einem oder mehreren der Ansprüche 1 bis 3, **dadurch gekennzeichnet, dass** bei der Messung der Lösegeschwindigtkeit in einer Standard-Apparatur nach Paddle USP 027 bei 37°C und einer Rührerdrehzahl von 75 Upm von 20mg des kristallinen Hydrats der Formel I in 1000ml einer 0,1%igen wässrigen Natriumdodecylsulfatlösung nach 15 Minuten bereits mindestens 50% des kristallinen Hydrats der Formel I in Lösung sind.

12. Kristallines Hydrat der Formel I, gemäß Anspruch 11, **dadurch gekennzeichnet, dass** nach 15 Minuten bereits mindestens 70% des kristallinen Hydrats der Formel I in Lösung sind.

13. Kristallines Hydrat der Formel I, gemäß Anspruch 11, **dadurch gekennzeichnet, dass** nach 15 Minuten bereits mindestens 85% des kristallinen Hydrats der Formel I in Lösung sind.

14. Arzneimittel enthaltend die Verbindung gemäß einem oder mehreren der Ansprüche 1 bis 13.

15. Arzneimittel enthaltend die Verbindung gemäß einem oder mehreren der Ansprüche 1 bis 13 und mindestens einen weiteren Wirkstoff.

16. Arzneimittel, gemäß Anspruch 15, **dadurch gekennzeichnet, daß** es als weiteren Wirkstoff eine oder mehrere Verbindungen, die den Lipidstoffwechsel normalisieren, enthält.

17. Arzneimittel, gemäß Anspruch 15 oder 16, **dadurch gekennzeichnet, daß** es als weiteren Wirkstoff eine oder mehrere Antidiabetika, hypoglykämischen Wirkstoffe, Antiadiposita, Anorektika, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, Cannabinoid Rezeptor 1 Antagonisten, CART-Agonisten, NPY-Agonisten, Cannabinoid Rezeptor 1 Antagonisten, MCH Rezeptor Antagonisten, MC4-Agonisten, Orexin-Agonisten, H3-Agonisten, TNF-Agonisten, CRF-Agonisten, CRF BP-Antagonisten, GLP-1-Derivate, Urocortin-Agonisten, β3-Agonisten, MSH (Melanocyt-stimulierendes Hormon)-Agonisten, CCK-A Agonisten, Serotonin-Wiederaufnahme-Inhibitoren, gemischte Sertonin-und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, 11β-HSD1-Hemmstoffe, ACC-Hemmstoffe, DPP-IV-Hemmstoffe, PPAR-Modulatoren, RXR-Modulatoren oder TR-β-Agonisten oder Amphetamine enthält.

18. Arzneimittel, gemäß Anspruch 17, **dadurch gekennzeichnet, daß** es als weiteren Wirkstoff eine oder mehrere Statine enthält.

19. Arzneimittel, gemäß Anspruch 18, **dadurch gekennzeichnet, daß** es als weiteren Wirkstoff Simvastatin, Fluvastatin, Pravastatin, Lovastatin, Atorvastatin, Cerivastatin oder Rosuvastatin enthält.

20. Verfahren zur Herstellung eines Arzneimittels enthaltend die Verbindung gemäß einem oder mehreren der Ansprüche 1 bis 13, **dadurch gekennzeichnet, daß** der Wirkstoff mit einem pharmazeutisch geeigneten Träger vermischt wird und diese Mischung in eine für die Verabreichung geeignete Form gebracht wird.

21. Verbindung gemäß einem oder mehreren der Ansprüche 1 bis 13 als Arzneimittel.

22. Verbindung gemäß einem oder mehreren der Ansprüche 1 bis 13 zur Behandlung von Lipidstoffwechselstörungen.

23. Verbindung gemäß einem oder mehreren der Ansprüche 1 bis 13 zur Behandlung von Hyperlipidämie.

24. Verbindung gemäß einem oder mehreren der Ansprüche 1 bis 13 zur Senkung des Serumcholesterinspiegels.

25. Verwendung der Verbindung gemäß einem oder mehreren der Ansprüche 1 bis 13 zur Herstellung eines Medikaments zur Behandlung von Lipidstoffwechselstörungen.

26. Verwendung der Verbindung gemäß einem oder mehreren der Ansprüche 1 bis 13 zur Herstellung eines Medikaments zur Behandlung von Hyperlipidämie.

27. Verwendung der Verbindung gemäß einem oder mehreren der Ansprüche 1 bis 13 zur Herstellung eines Medikaments zur Senkung des Serumcholesterinspiegels.

28. Verfahren zur Herstellung der kristallininen Hydrate der Formel I, worin n einen Wert von 0,5 bis 1,8 besitzt, **dadurch gekennzeichnet, dass** die amorphe Verbindung der Formel II 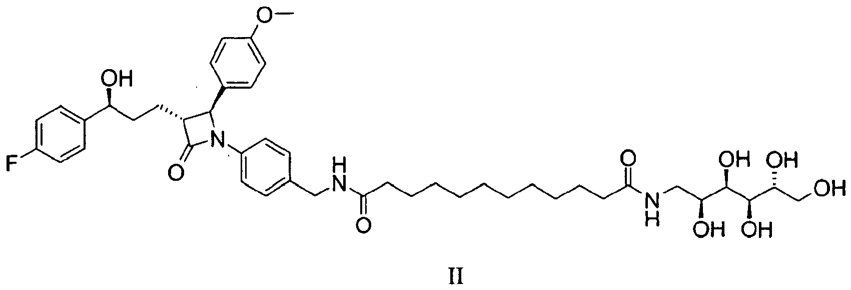 in einem organischen Lösungsmittel gelöst wird und diese Lösung zu einer Suspension von Impfkristallen der Verbindung der Formel I gegeben wird.

29. Verfahren nach Anspruch 28, **dadurch gekennzeichnet, dass** als organisches Lösungsmittel Ethanol oder eine Ethanol/Wasser Mischung verwendet wird.

30. Verfahren zur Herstellung der kristallinen Hydrate der Formel I gemäß einem oder mehreren der Ansprüche 1 bis 10, **dadurch gekennzeichnet, dass** reine Verbindung der Formel II 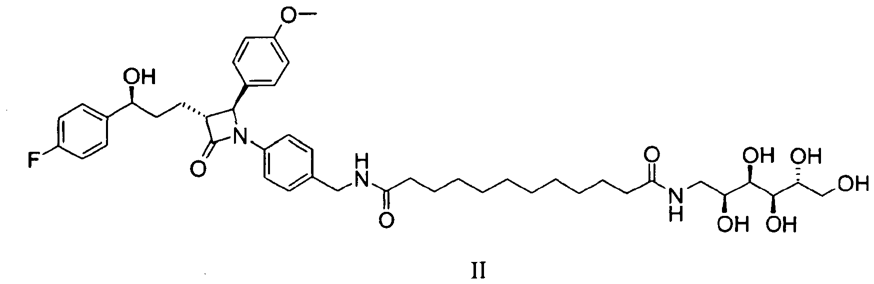 mit einem Gehalt von >99% mit Wasser ausgerührt wird.

## Claims

1. A crystalline hydrate of the formula I 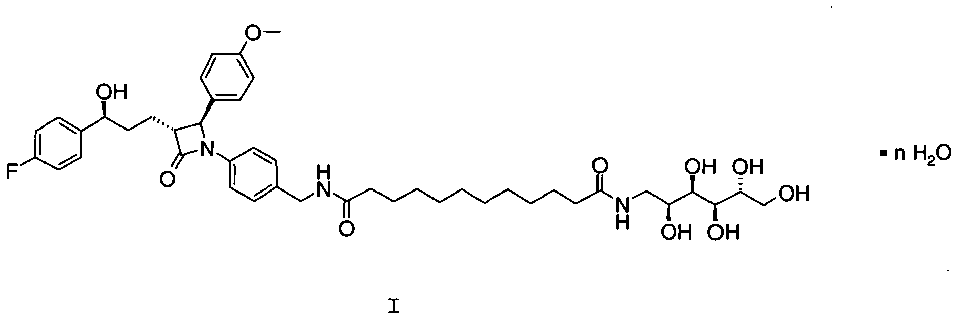 in which n has a value of from 0.5 to 1.8.

2. The crystalline hydrate of the formula I as claimed in claim 1, wherein n has a value of from 0.8 to 1.3.

3. The crystalline hydrate of the formula I as claimed in claim 1 or 2, wherein n has a value of from 1.0 to 1.2.

4. The crystalline hydrate of the formula I as claimed in one or more of claims 1 to 3, wherein the XRPD measured with CuKα radiation has a main peak of 20.83 degrees 2 theta ± 0.2 degree 2 theta.

5. The crystalline hydrate of the formula I as claimed in one or more of claims 1 to 3, wherein the XRPD measured with CuKα radiation has at least peaks of the following 2 theta values:
20.83 and 21.58 ± 0.2 degree 2 theta.

6. The crystalline hydrate of the formula I as claimed in one or more of claims 1 to 3, wherein the XRPD measured with CuKα radiation has at least peaks of the following 2 theta values:
12.19, 20.83 and 21.58 ± 0.2 degree 2 theta.

7. The crystalline hydrate of the formula I as claimed in one or more of claims 1 to 4, wherein the XRPD measured with CuKα radiation has at least peaks of the following 2 theta values:
20.43, 20.83 and 21.58 ± 0.2 degree 2 theta.

8. The crystalline hydrate of the formula I as claimed in claim 7, wherein the XRPD measured with CuKα radiation has at least peaks of the following 2 theta values:
12.19, 17.31, 20.43, 20.83 and 21.58 ± 0.2 degree 2 theta.

9. The crystalline hydrate of the formula I as claimed in one or more of claims 1 to 3, wherein the XRPD measured with CuKα radiation has at least peaks of the following 2 theta values:
7.33, 12.19, 16.05, 17.31, 20.83 and 21.58 ± 0.2 degree 2 theta.

10. The crystalline hydrate of the formula I as claimed in claim 8, wherein the XRPD measured with CuKα radiation has at least peaks of the following 2 theta values:
7.33, 8.92, 12.19, 16.05, 17.31, 17.68, 18.83, 20.43, 20.83, 21.58, 24.55, 25.37 ± 0.2 degree 2 theta.

11. The crystalline hydrate of the formula I as claimed in one or more of claims 1 to 3, wherein, in the measurement of the dissolution rate in a standard Paddle USP 027 apparatus at 37°C and a stirrer speed of 75 rpm, at least 50% of 20 mg of the crystalline hydrate of the formula I are already in solution in 1000 ml of a 0.1% aqueous sodium dodecylsulfate solution after 15 minutes.

12. The crystalline hydrate of the formula I as claimed in claim 11, wherein at least 70% of the crystalline hydrate of the formula I are already in solution after 15 minutes.

13. The crystalline hydrate of the formula I as claimed in claim 11, wherein at least 85% of the crystalline hydrate of the formula I are already in solution after 15 minutes.

14. A medicament comprising the compound as claimed in one or more of claims 1 to 13.

15. A medicament comprising the compound as claimed in one or more of claims 1 to 13 and at least one further active ingredient.

16. The medicament as claimed in claim 15, which comprises, as a further active ingredient, one or more compounds which normalize lipid metabolism.

17. The medicament as claimed in claim 15 or 16, which comprises, as a further active ingredient, one or more antidiabetics, active hypoglycemic ingredients, antiobesity agents, anorectics, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, lipase inhibitors, insulins, sulfonylureas, biguanides, meglitinides, thiazolidinediones, α-glucosidase inhibitors, active ingredients which act on the ATP-dependent potassium channel of the beta cells, cannabinoid receptor 1 antagonists, CART agonists, NPY agonists, cannabinoid receptor 1 antagonists, MCH receptor antagonists, MC4 agonists, orexin agonists, H3 agonists, TNF agonists, CRF agonists, CRF BP antagonists, GLP-1 derivatives, urocortin agonists, β3 agonists, MSH (melanocyte-stimulating hormone) agonists, CCK-A agonists, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptinagonists, DA agonists (bromocriptine, doprexin), lipase/amylase inhibitors, 11ß-HSD1 inhibitors, ACC inhibitors, DPP-IV inhibitors, PPAR modulators, RXR modulators or TR-β agonists or amphetamines.

18. The medicament as claimed in claim 17, which comprises, as a further active ingredient, one or more statins.

19. The medicament as claimed in claim 18, which comprises, as a further active ingredient, simvastatin, fluvastatin, pravastatin, lovastatin, atorvastatin, cerivastatin or rosuvastatin.

20. A process for producing a medicament comprising the compound as claimed in one or more of claims 1 to 13, which comprises mixing the active ingredient with a pharmaceutically suitable carrier and converting this mixture to a form suitable for administration.

21. The compound as claimed in one or more of claims 1 to 13 as a medicament.

22. The compound as claimed in one or more of claims 1 to 13 for treatment of lipid metabolism disorders.

23. The compound as claimed in one or more of claims 1 to 13 for treatment of hyperlipidemia.

24. The compound as claimed in one or more of claims 1 to 13 for lowering the serum cholesterol level.

25. The use of the compound as claimed in one or more of claims 1 to 13 for producing a medicament for treatment of lipid metabolism disorders.

26. The use of the compound as claimed in one or more of claims 1 to 13 for producing a medicament for treatment of hyperlipidemia.

27. The use of the compound as claimed in one or more of claims 1 to 13 for producing a medicament for lowering the serum cholesterol level.

28. A process for preparing the crystalline hydrates of the formula I in which n has a value of from 0.5 to 1.8, which comprises dissolving the amorphous compound of the formula II 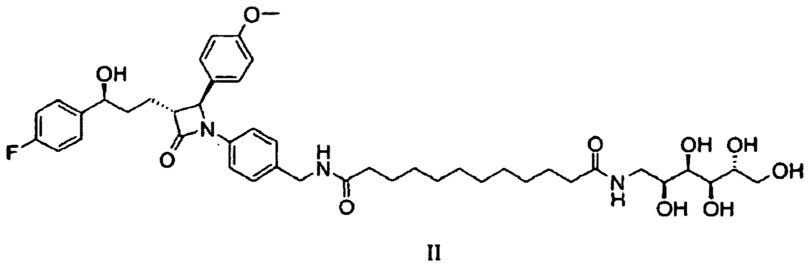 in an organic solvent and adding this solution to a suspension of seed crystals of the compound of the formula I.

29. The process as claimed in claim 28, wherein the organic solvent used is ethanol or an ethanol/water mixture.

30. A process for producing the crystalline hydrates of the formula I as claimed in one or more of claims 1 to 10, which comprises stirring pure compound of the formula II 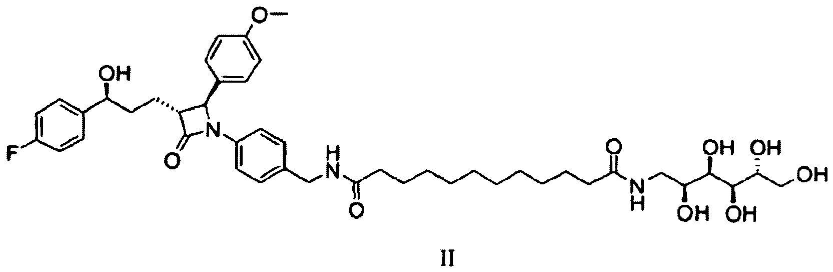 having a content of > 99% with water.

## Revendications

1. Hydrates cristallins de formule I, 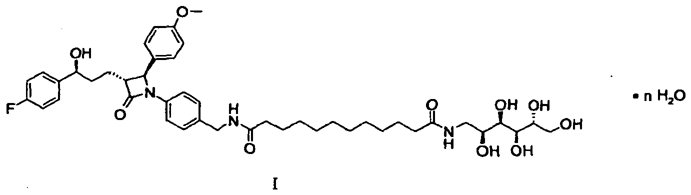 dans laquelle n présente une valeur de 0,5 à 1,8.

2. Hydrates cristallins de formule I, selon la revendication 1, **caractérisés en ce que** n présente une valeur de 0,8 à 1,3.

3. Hydrate cristallin de formule I, selon la revendication 1 ou 2, **caractérisé en ce que** n présente une valeur de 1,0 à 1,2.

4. Hydrate cristallin de formule I, selon l'une ou plusieurs des revendications 1 à 3, **caractérisé en ce que** la XRPD (diffraction de rayons X sur une poudre) mesurée à l'aide d'un rayonnement CuKα présente un pic principal de 20,83 degrés 2 Thêta ± 0,2 degré 2 Thêta.

5. Hydrate cristallin de formule I, selon l'une ou plusieurs des revendications 1 à 3, **caractérisé en ce que** la XRPD mesurée à l'aide d'un rayonnement CuKα présente au moins des pics des valeurs 2 Thêta suivantes : 20,83 et 21,58 ± 0,2 degrés 2 Thêta.

6. Hydrate cristallin de formule I, selon l'une ou plusieurs des revendications 1 à 3, **caractérisé en ce que** la XRPD mesurée à l'aide d'un rayonnement CuKα présente au moins des pics des valeurs 2 Thêta suivantes : 12,19, 20,83 et 21,58 ± 0,2 degrés 2 Thêta.

7. Hydrate cristallin de formule I, selon l'une ou plusieurs des revendications 1 à 4, **caractérisé en ce que** la XRPD mesurée à l'aide d'un rayonnement CuKα présente au moins des pics des valeurs 2 Thêta suivantes : 20,43, 20,83, 21,58 ± 0,2 degrés 2 Thêta.

8. Hydrate cristallin de formule I, selon la revendication 7, **caractérisé en ce que** la XRPD mesurée à l'aide d'un rayonnement CuKα présente au moins des pics des valeurs 2 Thêta suivantes : 12,19, 17,31, 20,43, 20,83, 21,58 ± 0,2 degrés 2 Thêta.

9. Hydrate cristallin de formule I, selon l'une ou plusieurs des revendications 1 à 3, **caractérisé en ce que** la XRPD mesurée à l'aide d'un rayonnement CuKα présente au moins des pics des valeurs 2 Thêta suivantes: 7,33, 12,19, 16,05, 17,31, 20,83, 21,58 ± 0,2 degrés 2 Thêta.

10. Hydrate cristallin de formule I, selon la revendication 8, **caractérisé en ce que** la XRPD mesurée à l'aide d'un rayonnement CuKα présente au moins des pics des valeurs 2 Thêta suivantes : 7,33, 8,92, 12,19, 16,05, 17,31, 17,68, 18,83, 20,43, 20,83, 21,58, 24,55, 25,37 ± 0,2 degrés 2 Thêta.

11. Hydrate cristallin de formule I, selon l'une ou plusieurs des revendications 1 à 3, **caractérisé en ce que** lors de la mesure de la vitesse de dissolution, dans un appareil standard selon Paddle USP 027 à 37°C et à une vitesse de l'agitateur de 75 t/min, de 20 mg de l'hydrate cristallin de formule I dans 1000 ml d'une solution aqueuse à 0,1% de dodécylsulfate de sodium, après 15 minutes, déjà au moins 50% de l'hydrate cristallin de formule I sont en solution.

12. Hydrate cristallin de formule I, selon la revendication 11, **caractérisé en ce qu'**après 15 minutes, déjà au moins 70% de l'hydrate cristallin de formule I sont en solution.

13. Hydrate cristallin de formule I, selon la revendication 11, **caractérisé en ce qu'**après 15 minutes, déjà au moins 85% de l'hydrate cristallin de formule I sont en solution.

14. Médicament contenant le composé selon l'une ou plusieurs des revendications 1 à 13.

15. Médicament contenant le composé selon l'une ou plusieurs des revendications 1 à 13 et au moins une autre substance active.

16. Médicament selon la revendication 15, **caractérisé en ce qu'**il contient, comme autre substance active, un ou plusieurs composés qui normalisent le métabolisme des lipides.

17. Médicament selon la revendication 15 ou 16, **caractérisé en ce qu'**il contient comme autre substance active un(e) ou plusieurs antidiabétiques, substances actives hypoglycémiantes, substances anti-adipeuses, substances anorexigènes, inhibiteurs de la HMGCoA-réductase, inhibiteurs de la résorption du cholestérol, agonistes de PPAR gamma, agonistes de PPAR alpha, agonistes de PPAR alpha/gamma, fibrates, inhibiteurs de MTP, inhibiteurs de la résorption de l'acide biliaire, inhibiteurs de CETP, adsorbants polymères de l'acide biliaire, inducteurs des récepteurs de LDL, inhibiteurs d'ACAT, antioxydants, inhibiteurs de la lipoprotéine-lipase, inhibiteurs de l'ATP-citrate-lyase, inhibiteurs de la squalène synthétase, antagonistes de la lipoprotéine (a), inhibiteurs de lipase, insulines, sulfonylurées, biguanides, méglitinides, thiazolidinediones, inhibiteurs de l'α-glucosidase, substances actives agissant sur le canal calcique dépendant de l'ATP des cellules bêta, antagonistes du récepteur 1 des cannabinoïdes, agonistes de CART, agonistes de NPY, antagonistes de récepteurs de MCH, agonistes de MC4, agonistes d'orexine, agonistes de H3, agonistes de TNF, agonistes de CRF, antagonistes de CRF BP, dérivés de GLP-1, agonistes d'urocortine, agonistes ß3, agonistes de MSH (hormone stimulant les mélanocytes), agonistes de CCK-A, inhibiteurs de la réabsorption de la sérotonine, composés sérotoninergiques et noradrénergiques mixtes, agonistes de 5HT, agonistes de bombésine, agonistes de galanine, hormones de croissance, composés libérant l'hormone de croissance, agonistes de TRH, modulateurs de la protéine découplante 2 ou 3, agonistes de leptine, agonistes de DA (bromocriptine, doprexine), inhibiteurs de lipase/amylase, inhibiteurs de 11B-HSD1, inhibiteurs d'ACC, inhibiteurs de DPP-IV, modulateurs de PPAR, modulateurs de RXR ou agonistes de TR-ß ou amphétamines.

18. Médicament selon la revendication 17, **caractérisé en ce qu'**il contient comme autre substance active une ou plusieurs statines.

19. Médicament selon la revendication 18, **caractérisé en ce qu'**il contient comme autre substance active la simvastatine, la fluvastatine, la pravastatine, la lovastatine, l'atorvastatine, la cérivastatine ou la rosuvastatine.

20. Procédé pour la préparation d'un médicament contenant le composé selon l'une ou plusieurs des revendications 1 à 13, **caractérisé en ce que** la substance active est mélangée avec un support pharmaceutiquement approprié et ce mélange est amené dans une forme appropriée pour l'administration.

21. Composé selon l'une ou plusieurs des revendications 1 à 13 en tant que médicament.

22. Composé selon l'une ou plusieurs des revendications 1 à 13 destiné au traitement de troubles du métabolisme des lipides.

23. Composé selon l'une ou plusieurs des revendications 1 à 13 destiné au traitement de l'hyperlipidémie.

24. Composé selon l'une ou plusieurs des revendications 1 à 13 destiné à abaisser le niveau de cholestérol dans le sérum.

25. Utilisation du composé selon l'une ou plusieurs des revendications 1 à 13 pour la préparation d'un médicament destiné au traitement de troubles du métabolisme des lipides.

26. Utilisation du composé selon l'une ou plusieurs des revendications 1 à 13 pour la préparation d'un médicament destiné au traitement de l'hyperlipidémie.

27. Utilisation du composé selon l'une ou plusieurs des revendications 1 à 13 pour la préparation d'un médicament destiné à abaisser le niveau de cholestérol dans le sérum.

28. Procédé pour la préparation des hydrates cristallins de formule I, dans laquelle n présente une valeur de 0,5 à 1,8, **caractérisé en ce que** le composé amorphe de formule II 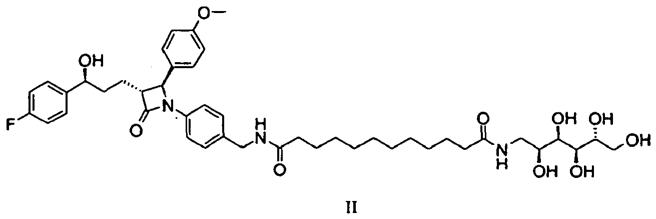 est dissous dans un solvant organique et cette solution est ajoutée à une suspension de cristaux d'ensemencement du composé de formule I.

29. Procédé selon la revendication 28, **caractérisé en ce qu'**on utilise, comme solvant organique, de l'éthanol ou un mélange éthanol/eau.

30. Procédé pour la préparation des hydrates cristallins de formule I selon l'une ou plusieurs des revendications 1 à 10, **caractérisé en ce que** le composé pur de formule II 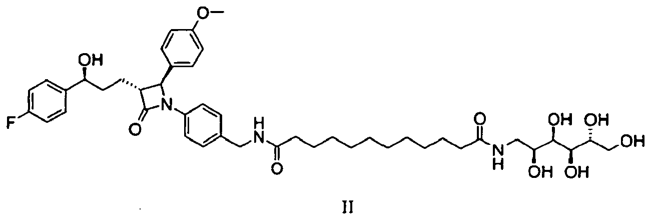 est agité à une teneur > 99% avec de l'eau.
